# EUROPEAN PATENT APPLICATION

(11) **EP 2 495 255 A1**
(43) Date of publication of application: **05.09.2012**
(21) Application number: 10826655.2
(22) Date of filing: 25.10.2010
(51) Int. Cl.: C07K 14/605, A61K 38/26, A61P 1/04, A61P 1/14, A61P 3/04, A61P 3/10, A61P 9/00, A61P 9/10

(54) **GLYCOSYLATED FORM OF ANTIGENIC GLP-1 ANALOGUE**

(30) Priority: 30.10.2009 JP 2009249597
(71) Applicant: Otsuka Chemical Co., Ltd., Osaka-shi, Osaka 540-0021 (JP)
(72) Inventor: KAJIHARA, Yasuhiro, Toyonaka-shi Osaka 560-0054 (JP); TSUJI, Takashi, Nagareyama-shi Chiba 270-0111 (JP); NAMBU, Yuri, Tokushima-shi Tokushima 771-0093 (JP); ISHII, Kazuyuki, Tokushima-shi Tokushima 771-0093 (JP); YOSHIDA, Kenta, Tokushima-shi Tokushima 771-0093 (JP); TEZUKA, katsunari, Tokushima-shi Tokushima 771-0093 (JP)
(74) Representative: Wasner, Marita
(86) International application number: PCT/JP2010/068814
(87) International publication number: WO 2011/052523

(57) **Abstract**

Disclosed is a GLP-1 analogue which is obtained by ameliorating a highly antigenic GLP-1 analogue so that the GLP-1 analogue has reduced antigenicity without being lowered in the blood glucose suppressing activity. Specifically disclosed is a glycosylated form of an antigenic GLP-1 analogue, which has GLP-1 activity and is obtained by substituting at least one amino acid of an antigenic GLP-1 analogue with a glycosylated amino acid.

## Description

### Technical Field

The present invention relates to an oligosaccharide chain added form of antigenic GLP-1 analogue, wherein a oligosaccharide chain is added to those having high antigenicity among GLP-1 analogues.

### Background Art

GLP-1 (glucagon-like peptide-1) is a peptide of intestinal origion deeply involved in the control of sugar homeostasis. GLP-1 is synthesized in L-cells of the intestines by tissue-specific post-translational processing of a glucagon precursor prepro-glucagon, which reacts to diet and released into circulation. This peptide is the primary mediator of the enteroinsular axis, and acts by binding to a particular receptor.

GLP-1 is known to mainly act on the pancreas to promote insulin release by β-cells in a glucose concentration-dependent manner. It is also suggested that it may suppress glucagon excretion, delay hollowing of the stomach, and increase peripheral glucose processing.

Since administration of GLP-1 may normalize postprandial glucose level in insulin-independent diabetes patients, the possibility of GLP-1 as a therapeutic drug is suggested. GLP-1 also has an effect of improving blood glucose control in insulin-dependent diabetes patients. Further, since insulin release promoting action of GLP-1 is dependent on plasma glucose concentration, GLP-1-mediated insulin release is low at low plasma glucose concentrations, and there is an advantage of not causing severe hypoglycemia. Accordingly, it is thought that a highly safe diabetes therapy will be possible by controlling blood GLP-1 amount as necessary. However, blood half-life of GLP-1 is extremely short at 2 to 6 minutes, and there is a problem that its potential as a therapeutic agent will be limited.

There is an attempt to modify GLP-1 as a means to solve these problems. For example, Patent Document 1 discloses a PEGylated GLP-1 compound comprising GLP-1 compound to which at least one polyethylene glycol (PEG) molecule is coupled. In such a PEGylated GLP-1 compound, each PEG is bound to the GLP-1 compound at Cys or Lys amino acid, or at the carboxy-terminal amino acid. The PEGylated GLP-1 compound has an excretion half-life of at least one hour.

According to Patent Document 1, a bioactive peptide having extended half-life and delayed clearance relative to an unPEGylated peptide is obtained. These PEGylated GLP-1 compounds and compositions are also disclosed to be useful in health status therapies such as diabetes, obesity, irritable intestines syndrome, as well as reducing blood glucose, suppressing gastric and/or intestinal motility, and suppressing gastric and/or intestinal content excretion or suppressing food intake (e.g. Non-Patent Document 1).

However, because PEG is a compound that is not metabolized in vivo, continued administration of a PEGylated GLP-1 compound will lead to in vivo accumulation of PEG, causing a risk of drug-induced sufferings in the organism (Non-Patent Document 1).

A method of adding an oligosaccharide chain onto GLP-1 or a modified form thereof to extend its half-life has also been proposed (e.g. Patent Documents 2 and 3). On the other hand, Patent Document 2 describes a method of binding a hyaluronic acid modification having a molecular weight of about 200 KDa to the GLP-1 analogue. However, when manufacturing such an enormous hyaluronic acid molecule in large amounts, it is difficult to have homogeneous lengths or structures, and it is thought that a substantial variation in the structure or length of each hyaluronic acid will occur in effect. When employed as a pharmaceutical, an oligosaccharide chain added peptide having homogeneous length or structure is required. On the other hand, Patent Document 3 describes e.g. a method for introducing oligosaccharide chain added amino acids at positions 26, 34, and/or 37 of GLP-1, but it cannot be said that the type of oligosaccharide chain or the position for adding the oligosaccharide chain are necessarily optimized.
The present inventors have also altered the type of oligosaccharide chain or the position for addition to develop an oligosaccharide chain added GLP-1 having long blood half-life and high activity (e.g. Patent Document 4).

Meanwhile, exendin-4, which was found from lizard (Heloderma) saliva (Non-Patent Document 2), is commercialized in the United States as a compound having a structure similar to GLP-1, having similar activity, and having high blood stability. However, since exendin-4 is a non-human sequence, its antigenicity is higher than other GLP-1 analogues, and emergence of neutralizing antibodies due to long-term administration or the accompanying reduction of drug action is concerned (Non-Patent Documents 3 to 5).

Liraglutide, which is GLP-1 bound to a fatty acid, has also been devoloped as one of GLP-1 analogues (see e.g. Non-Patent Document 6 to 8). Binding of a fatty acid increases affinity with albumin. Because liraglutide bound to albumin is slowly released into the blood, its half-life will be approximately 10 hours and a long-term action is anticipated. It is also highly convenient since once daily subcutaneous injection will suffice. Not only combination therapy, but single-agent therapy is also thought to be possible.
However, antigenicity is concerned as with exendin-4 since it has a structure different from the natural form. In particular, since about 99% of the administered amount of liraglutide binds to albumin, the administeration amount of liraglutide is raised, and in this perspective also, the antigenicity of the compound must be reduced as much as possible.
Patent Document 1
   National Publication of PCT International Application No. 2006-520818
Patent Document 2
   National Re-publication of PCT International Application No. 2006-095775
Patent Document 3
   International Publication No. 2007/063907
Patent Document 4
   International Publication No. 2008/155900
Non-Patent Document 1
   Bendele, A. et .al.: Toxicological Science, 1998, 42: 152-157
Non-Patent Document 2
   Journal of Biological Chemistry, 1992, 267: 7402-7405
Non-Patent Document 3
   Schnabel, C.A. et al.: Vascular Health and Risk Management, 2006, 2: 69-77
Non-Patent Document 4
   Amori, R.E. et al.: The Journal of the American Medical Association, 2007, 298: 194-206
Non-Patent Document 5
   Wajchenberg, B.L.: Endocrine Reviews, 2007, 28: 187-218
Non-Patent Document 6
   Marre, M. et al.: Diabetic Medicine, 2009, 26 (3): 268-278
Non-Patent Document 7
   Deacon, C.F.: Vascular Health and Risk Management, 2009, 5: 199-211
Non-Patent Document 8
   Larsen, P.J. et al.: Diabetes, 2001, 50: 2530-2539

### Disclosure of the Invention

### Problems to be Solved by the Invention

The object of the present invention is to provide a GLP-1 analogue which is obtained by improving highly antigenic GLP-1 analogue so that the GLP-1 analogue has reduced antigenicity without being lowered in the blood glucose suppressing activity.

### Means for Solving the Problems

The present inventors, as a resulted of repeated investigation to solve the above problems, found that by adding a oligosaccharide chain to a GLP-1 analogue having antigenicity such as exendin-4, antigenicity is reduced without being lowered in the blood glucose suppressing activity, and thus completed the present invention.
In other words, the present invention relates to:
[1] an oligosaccharide chain added form of antigenic GLP-1 analogue having GLP-1 activity, wherein at least one amino acid of the antigenic GLP-1 analogue is substituted with an oligosaccharide chain added amino acid;
[2] the oligosaccharide chain added form of antigenic GLP-1 analogue according to the above [1], wherein said oligosaccharide chain added amino acid is an oligosaccharide chain added Asn or an oligosaccharide chain added Cys;
[3] the oligosaccharide chain added form of antigenic GLP-1 analogue according to the above [1] or [2], wherein the oligosaccharide chain and the amino acid are bound via a linker in said oligosaccharide chain added amino acid;
[4] the oligosaccharide chain added form of antigenic GLP-1 analogue according to any one of the above [1] to [3], wherein said oligosaccharide chain is a oligosaccharide chain consisting of 4 or more sugars;
[5] the oligosaccharide chain added form of antigenic GLP-1 analogue according to any one of the above [1] to [4], wherein said oligosaccharide chain is a double-stranded complex oligosaccharide chain;
[6] the oligosaccharide chain added form of antigenic GLP-1 analogue according to the above [5], wherein said oligosaccharide chain is a oligosaccharide chain selected from disialooligosaccharide chain, monosialooligosaccharide chain, asialooligosaccharide chain, diGlucNAc oligosaccharide chain, and dimannose oligosaccharide chain;
[7] the oligosaccharide chain added form of antigenic GLP-1 analogue according to the above [5], wherein said oligosaccharide chain is an oligosaccharide chain represented by: 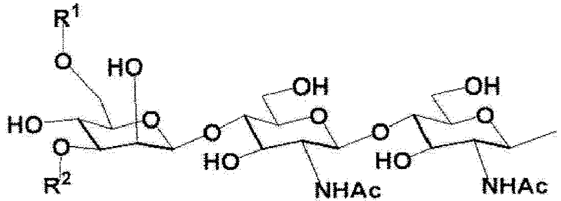 [wherein R¹ and R² are the same or different, and indicate: 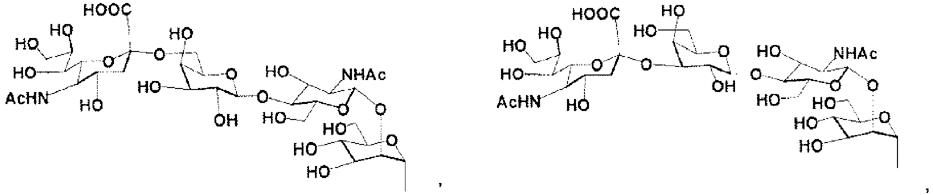 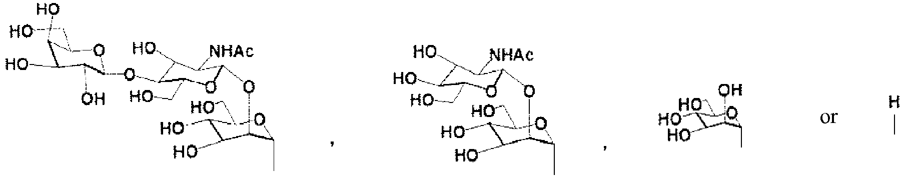 and Ac indicates an acetyl group];
[8] the oligosaccharide chain added form of antigenic GLP-1 analogue according to any one of the above [1] to [7], wherein said antigenic GLP-1 analogue is exendin-4;
[9] the oligosaccharide chain added form of antigenic GLP-1 analogue according to the above [8], wherein the site substituted with an oligosaccharide chain added amino acid is position 30 in the amino acid sequence set forth in SEQ ID NO: 2;
[10] the oligosaccharide chain added form of antigenic GLP-1 analogue according to the above [8], wherein:
   in the peptide consisting of the amino acid sequence set forth in SEQ ID NO: 2, Gly at position 30 is substituted to oligosaccharide chain added Cys,
   said oligosaccharide chain is the disialooligosaccharide chain shown by the following formula: 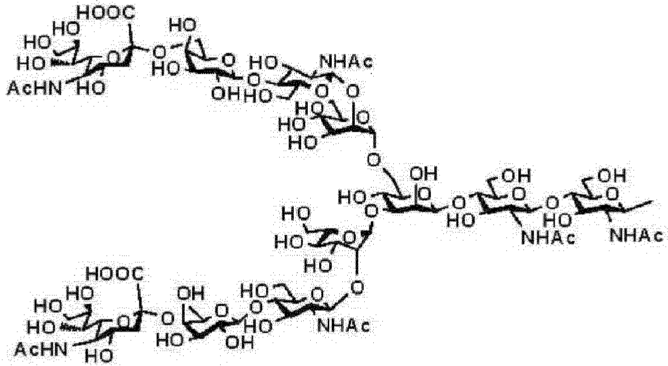 and
      said oligosaccharide chain and said Cys are bound via a linker in said oligosaccharide chain added Cys;
[11] the oligosaccharide chain added form of antigenic GLP-1 analogue according to any one of the above [1] to [7], wherein said antigenic GLP-1 analogue is liraglutide;
[12] the oligosaccharide chain added form of antigenic GLP-1 analogue according to the above [11], wherein said site substituted with a oligosaccharide chain added amino acid is position 30 in the amino acid sequence set forth in SEQ ID NO: 3;
[13] the oligosaccharide chain added form of antigenic GLP-1 analogue according to the above [11], wherein:
   in the peptide consisting of the amino acid sequence set forth in SEQ ID NO: 3, Arg at position 30 is substituted to oligosaccharide chain added Cys,
   said oligosaccharide chain is the disialooligosaccharide chain represented by the following formula: 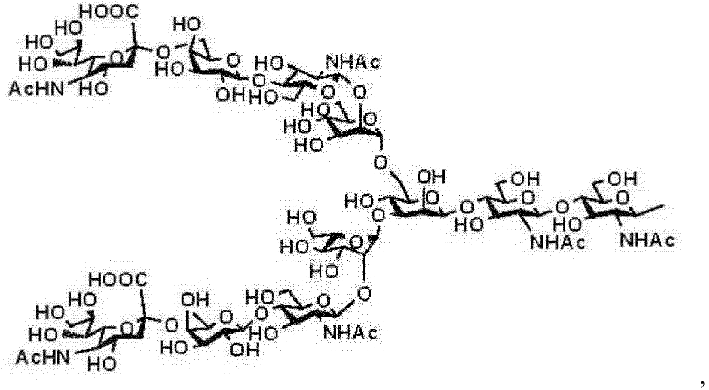 or the asialooligosaccharide chain represented by the following formula: 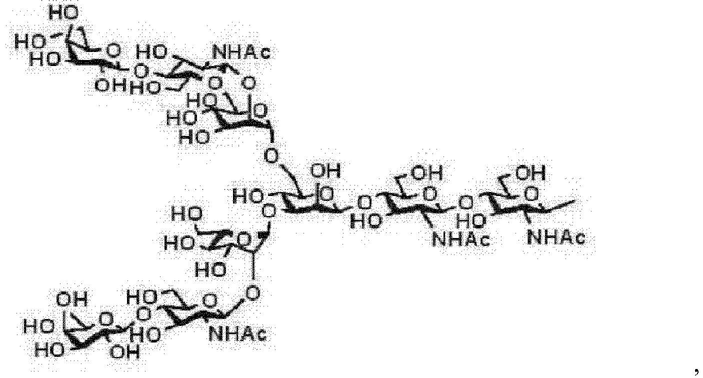 and
      said oligosaccharide chain and said Cys are bound via a linker in said oligosaccharide chain added Cys;
[14] the oligosaccharide chain added form of antigenic GLP-1 analogue according to any one of the above [1] to [13], wherein said oligosaccharide chain is substantially homogeneous;
[15] the oligosaccharide chain added form of antigenic GLP-1 analogue according to any one of the above [1] to [13], wherein said oligosaccharide chain is 99% or more homogeneous;
[16] the oligosaccharide chain added form of antigenic GLP-1 analogue according to any one of the above [1] to [15], wherein the antigenicity is half or less relative to an antigenic GLP-1 analogue without any oligosaccharide chains added thereto;
[17] the oligosaccharide chain added form of antigenic GLP-1 analogue according to any one of the above [1] to [16], wherein the antigenicity is reduced compared to the antigenic GLP-1 analogue without any oligosaccharide chains added thereto, and GLP-1 activity is elevated compared to natural form GLP-1;
[18] a pharmaceutical composition comprising the oligosaccharide chain added form of antigenic GLP-1 analogue according to any one of the above [1] to [17] as an active ingredient;
[19] the pharmaceutical composition according to the above [18] for therapy or prevention of a GLP-1-related disease;
[20] the pharmaceutical composition according to the above [19], wherein said GLP-1-related disease is diabetes;
[21] a therapeutic or prophylactic method of a GLP-1-related disease, characterized in adminstering an effective amount of the oligosaccharide chain added form of antigenic GLP-1 analogue according to any one of the above [1] to [17].

### Advantages of the Invention

The oligosaccharide chain added form of antigenic GLP-1 analogue of the present invention can be reduced in antigenicity without being lowered in the blood glucose suppressing activity by adding an oligosaccharide chain. Accordingly, a safe pharmaceutical that maintains the superiority of exendin-4 of having high blood stability as well as high blood glucose suppressing activity, or the superiority of liraglutide of allowing a fewer number of administrations by controlled release can be provided.
Since the oligosaccharide chain to be added is easily degraded in vivo, there are no drug-induced sufferings in the organism due to its accumulation.
Further, since many oligosaccharide chains used in the present invention are relatively short, those having homogeneous structure can be obtained without going through complex manufacturing steps. Accordingly, high-quality oligosaccharide chain added form of antigenic GLP-1 analogues of pharmaceutical level can be stably obtained in large scales.

### Brief Description of the Drawings

[Figure 1] Figure 1 shows an HPLC chromatogram of position 30 Cys-asialooligosaccharide chain added liraglutide (SEQ ID NO: 8);
[Figure 2] Figure 2 shows an HPLC chromatogram of a peptide having Arg at position 30 of liraglutide substituted with Cys (SEQ ID NO: 14) in Example 4;
[Figure 3] Figure 3 show the purification chromatogram by HPLC of position 30 Cys-disialooligosaccharide chain added liraglutide (SEQ ID NO: 9);
[Figure 4] Figure 4 shows the evaluation result of the antigenicity-lowering action of exendin-4 by glycosylation. When mice were sensitized with oligosaccharide chain added and non-oligosaccharide chain added exendin-4, the antibody titer of oligosaccharide chain added exendin-4 was about one-quarter compared to non-oligosaccharide chain added exendin-4;
[Figure 5] Figure 5 shows the result of oral glucose tolerance test (OGTT) of oligosaccharide chain added exendin-4. Oligosaccharide chain added exendin-4 showed blood glucose elevation suppressive action equal to non-oligosaccharide chain added exendin-4;
[Figure 6] Figure 6 shows the evaluation result of the blood glucose lowering action of oligosaccharide chain added exendin-4 using db/db mice. Oligosaccharide chain added exendin-4 showed strong blood glucose lowering action equal to non-oligosaccharide chain added exendin-4;
[Figure 7] Figure 7 shows the result of oral glucose tolerance test (OGTT) of oligosaccharide chain added liraglutide. Oligosaccharide chain added liraglutide showed blood glucose elevation suppressive action even higher than non-oligosaccharide chain added liraglutide; and
[Figure 8] Figure 8 shows the evaluation result of the blood glucose lowering action of oligosaccharide chain added liraglutide using db/db mice. Oligosaccharide chain added liraglutide showed blood glucose lowering action and sustainability equal to non-oligosaccharide chain added liraglutide.

### Best Mode for Carrying Out the Invention

"GLP-1" herein indicates glucagon-like peptide-1, and refers to GLP-1 (7-37).

GLP-1 (7-37) has the following amino acid sequence:

His-Ala-Glu-Gly-Thr-Phe-Thr-Ser-Asp-Val-Ser-Ser-Tyr-Leu-Glu-Gly-Gln-Ala-Ala-Lys-Glu-
Phe-Ile-Ala-Trp-Leu-Val-Lys-Gly-Arg-Gly (SEQ ID NO: 1).

"GLP-1 analogue" herein refers to a peptide having structure similar to GLP-1 and/or a peptide having structure overlapping GLP-1, for example: a peptide having one or more amino acids deleted, substituted, or added from GLP-1; a peptide having one or more amino acids conservatively substituted from the amino acids of GLP-1; a modified form of GLP-1; a fragment of GLP-1 having GLP-1 activity; and an elongated GLP-1 having GLP-1 activity etc.

An "antigenic GLP-1 analogue" herein refers to one having antigenicity among GLP-1 analogues. Antigenicity means antigenicity in an individual to which such antigenic GLP-1 analogue is administered, regardless of its extent. Antigenicity can be evaluated with methods well-known to those skilled in the art or methods corresponding thereto, examples of which include, e.g., a method of sensitizing a mouse with an antigenic GLP-1 analogue, and measuring the anti-GLP-1 analogue antibody titer in the blood of such mouse.
Antigenic GLP-1 analogues include, but are not limited to, e.g., exendin-4 (hereinafter may be described as "Ex-4"), liraglutide, taspoglutide (otherwise known as BIM-51077, Giannoukakis, N.: Curr. Opin. Investig. Drugs, 2007, 8:842-848), and ZP-10A (Thorkildsen, C. et al.: J. Pharmacol. Exp. Ther., 2003, 307:490-496) etc.

"Exendin-4" herein is, unless otherwise indicated, in addition to the peptide consisting of the amino acid sequence set forth in SEQ ID NO: 2, a peptide having structure similar to such peptide and/or a peptide having structure overlapping such peptide, for example: a peptide having one or more amino acids deleted, substituted, or added from such peptide; a peptide having one or more amino acids conservatively substituted from the amino acids of such peptide; a modified form of such peptide; a fragment of such peptide having activity equal to exendin-4; and a peptide elongated from such peptide having activity equal to exendin-4 etc., including those corresponding to the above GLP-1 analogues.

Further, "liraglutide" herein is, unless otherwise indicated, in addition to the peptide consisting of the amino acid sequence set forth in SEQ ID NO: 3, a peptide having structure similar to such peptide and/or a peptide having structure overlapping such peptide, for example: a peptide having one or more amino acids deleted, substituted, or added from such peptide; a peptide having one or more amino acids conservatively substituted from the amino acids of such peptide; a modified form of such peptide; a fragment of such peptide having activity equal to liraglutide; and a peptide elongated from such peptide having activity equal to liraglutide etc., including those corresponding to the above GLP-1 analogues.

"BIM-51077" herein is, unless otherwise indicated, in addition to the peptide consisting of the amino acid sequence set forth in SEQ ID NO: 18, a peptide having structure similar to such peptide and/or a peptide having structure overlapping such peptide, for example: a peptide having one or more amino acids deleted, substituted, or added from such peptide; a peptide having one or more amino acids conservatively substituted from the amino acids of such peptide; a modified form of such peptide; a fragment of such peptide having activity equal to liraglutide; and a peptide elongated from such peptide having activity equal to liraglutide etc., including those corresponding to the above GLP-1 analogues.

An "amino acid" herein is used in its broadest sense, and includes not only natural amino acids but also non-natural amino acids such as amino acid variants and derivatives. Those skilled in the art, in light of this broad definition, will recognize that amino acids herein can include, e.g., natural proteinogenic L-amino acids; D-amino acids; chemically modified amino acids such as amino acid variants and derivatives; natural non-proteinogenic amino acids such as norleucine, β-alanine, and ornithine; and chemically synthesized compounds having properties which is characteristic of amino acids and is well-known in the art characteristic of amino acids etc. Examples of non-natural amino acids include α-methylamino acids (such as α-methylalanine), D-amino acids, histidine-like amino acids (such as 2-amino-histidine, β-hydroxy-histidine, homohistidine, α-fluoromethyl-histidine, and α-methyl-histidine), amino acids having excess methylene on the side chain ("homo" amino acids), and amino acids having the carboxylic acid functionality of the amino acid in the side chain substituted with a sulfonic group (such as cysteic acid). Several of antigenic GLP-1 analogues are known to comprise non-natural amino acids. In a preferred aspect, amino acids comprised in the compound of the present invention consist only of natural amino acids.

When referrering herein to "having one or more amino acids deleted, substituted, or added," the number of amino acids substituted etc. is not particularly limited as long as the oligosaccharide chain added form of the antigenic GLP-1 analogue of the present invention retains GLP-1 activity, but is 1 to 9, preferably 1 to 5, more preferably about 1 to 3, or within 20%, preferably within 10% of the total length. The substituted or added amino acids may be natural amino acids, non-natural amino acids, or amino acid analogues, preferably natural amino acids.

"Having one or more amino acids of the amino acid conservatively substituted" herein refers to an amino acid substitution in which the hydrophilic index and/or hydrophobic index of the original amino acid and the substituted amino acid are similar, and evident reduction or dissappearance of GLP-1 activity does not occur before and after such substitution.

The "modified form" of GLP-1, Ex-4, and liraglutide (hereinafter generically referred to as "GLP-1 etc.") herein is a naturally or artificially modified compound of GLP-1 etc., and such modification includes, e.g., alkylation, acylation (e.g. acetylation), amidation, carboxylation, ester formation, disulfide bond formation, glycosylation, lipidation, phosphorylation, hydroxylation, binding of a labelling component etc. of one or more amino acid residues of GLP-1 etc. For example, a peptide having the C-terminal amidated is also included.

A "fragment of GLP-1 etc. having GLP-1 activity" herein is GLP-1 etc. having one or more amino acids deleted from the N-terminal and/or C-terminal of the original GLP-1 etc., and maintains GLP-1 activity.

An "elongated peptide having GLP-1 activity" herein is a peptide having one or more amino acids added to the N-terminal and/or C-terminal of the original GLP-1 etc., and maintains GLP-1 activity (see e.g. Endocrinology, 125, 3109-14 (1989)).

An "oligosaccharide chain added form of antigenic GLP-1 analogue (glycosylated form of antigenic GLP-1 analogue)" herein is characterized in that at least one amino acid is substituted with an oligosaccharide chain added amino acid in the antigenic GLP-1 analogue. The "oligosaccharide chain added form of antigenic GLP-1 analogue" may be hereinafter referred to as "oligosaccharide chain added antigenic GLP-1."

The oligosaccharide chain added antigenic GLP-1 analogue of the present invention also comprises salts thereof. As used herein, salts may be either acid addition salts or base addition salt. Acids typically employed for formation of acid addition salts are inorganic acids such as hydrochloric acid, hydrobromic acid, hydroiodic acid, sulfuric acid, and phosphoric acid, and organic acids such as p-toluenesulfonic, methanesulfonic, oxalic, p-bromophenylsulfonic, carboxylic, succinic, citric, benzoic, and acetic acids. Base addition salts include salts derived from inorganic bases such as ammonium hydroxide or alkaline or alkaline earth hydroxide, carbonates, and bicarbonates. In particular, pharmceutically acceptable salts are preferred.

An "oligosaccharide chain added amino acid" herein is an amino acid to which an oligosaccharide chain is bound, wherein the oligosaccharide chain and the amino acid may be bound via a linker. The binding site between the oligosaccharide chain and the amino acid is not particularly limited, but it is preferable that the amino acid is bound to the reducing terminal of the oligosaccharide chain.
The type of amino acid to which the oligosaccharide chain binds is not particularly limited, and any of natural amino acids and non-natural amino acids can be used. In terms of having a structure same or similar to those in which oligosaccharide chain added amino acids exist in vivo as glycopeptides (glycoproteins), oligosaccharide chain added amino acid is preferably an N-linked oligosaccharide chain such as oligosaccharide chain added Asn, an O-linked oligosaccharide chain such as oligosaccharide chain added Ser and oligosaccharide chain added Thr, particularly preferably oligosaccharide chain added Asn.

In addition, in terms of ease of binding with a linker when the oligosaccharide chain and the amino acid are bound via a linker, the amino acid of the oligosaccharide chain added amino acid is preferably an amino acid having two or more carboxyl groups within the molecule such as aspartic acid or glutamic acid, an amino acid having two or more amino groups within the molecule such as lysine, arginine, histidine, tryptophan, an amino acid having a hydroxyl group within the molecule such as serine, threonine, tyrosine, an amino acid having a thiol group within the molecule such as cystein, and an amino acid having an amido group within the molecule such as asparagine, glutamine. In particular, in terms of reactivity, aspartic acid, glutamic acid, lysine, arginine, serine, threonine, cystein, asparagine, glutamine are preferred, and cystein and lysine are particularly preferred.

For any oligosaccharide chain added antigenic GLP-1 analogue of the present invention, when the oligosaccharide chain structure, structures other than the oligosaccharide chain, oligosaccharide chain addition sites, and the number of oligosaccharide chains added are identical, there is no large difference seen in blood glucose elevation suppression activity of the oligosaccharide chain added antigenic GLP-1 analogue of the present invention between those having oligosaccharide chain added Asn (not through a linker) and oligosaccharide chain added Cys (via a linker) as the oligosaccharide chain added amino acid.

When the oligosaccharide chain and the amino acid are bound via a linker, those employed in the corresponding field can be broadly used as the linker, and can include, e.g., - NH-(CO)-(CH₂)ₐ CH₂- (wherein a is an integer, and is not particularly limited as long as it does not inhibit target linker function, but preferably indicates an integer between 0 and 4), C₁₋₁₀ polymethylene, -CH₂-R- (wherein R is a group generated by one hydrogen atom detached from a group selected from the group consisting of alkyl, substituted alkyl, alkenyl, substituted alkenyl, alkynyl, substituted alkynyl, aryl, substituted aryl, carbocyclic group, substituted carbocyclic group, heterocyclic group, and substituted heterocyclic group), and - (CO)-(CH₂)ₐ-(CO)- (wherein a is an integer, and is not particularly limited as long as it does not inhibit target linker function, but preferably indicates an integer between 0 and 4) etc.

In the oligosaccharide chain added amino acid of the oligosaccharide chain added antigenic GLP-1 analogue, when the oligosaccharide chain and the amino acid are bound without being mediated by a linker, the antigenicity of the oligosaccharide chain added antigenic GLP-1 analogue may be reduced compared to when the oligosaccharide chain and the amino acid are bound via a linker. In the oligosaccharide chain added amino acid of the oligosaccharide chain added antigenic GLP-1 analogue, when the oligosaccharide chain and the amino acid are bound via a linker, the blood stability of the oligosaccharide chain added antigenic GLP-1 analogue may be higher compared to when the oligosaccharide chain and the amino acid are bound without being mediated by a linker.

The oligosaccharide chain added antigenic GLP-1 analogue of the present invention is not in any way limited in the manufacturing method by the description thereof (e.g. the description "a oligosaccharide chain added antigenic GLP-1 analogue having an amino acid substituted with a oligosaccharide chain added amino acid,") and oligosaccharide chain added antigenic GLP-1 analogues manufactured by any of methods A to C described below are included in "a oligosaccharide chain added antigenic GLP-1 analogue having an amino acid substituted with a oligosaccharide chain added amino acid." In addition, for example, a oligosaccharide chain added antigenic GLP-1 analogue having a oligosaccharide chain without any amino acids bound thereto, bound directly or via a linker to an amino acid on a peptide; a oligosaccharide chain added antigenic GLP-1 analogue wherein the already added oligosaccharide chain is elongated by further adding a sugar or oligosaccharide chain to the added oligosaccharide chain; and a oligosaccharide chain added antigenic GLP-1 analogue wherein one or more amino acids are bound to the amino group and/or carboxyl group of the oligosaccharide chain added amino acid, and further linking this to one or more fragments of the GLP-1 analogue etc. are also included in the oligosaccharide chain added antigenic GLP-1 analogue of the present invention, as long as the final structure match.

The number of substituions of the amino acid of the antigenic GLP-1 analogue with oligosaccharide chain added amino acids may be appropriately adjusted depending on physiological activity such as blood stability or blood glucose suppressing activity, the number of amino acids present in the final oligosaccharide chain added antigenic GLP-1 analogue, or the molecular weight of the oligosaccharide chain added antigenic GLP-1 analogue before and after glycosylation etc. For example, 1 to 5 substitutions are preferred, and more preferably 1 to 3 substitutions. In terms of convenience, if the desired activity is obtained by one substitution, it will be preferable to select one substitution. In general, in a oligosaccharide chain added antigenic GLP-1 analogue having one amino acid of the antigenic GLP-1 analogue substituted with a oligosaccharide chain added amino acid, if one or more amino acids other than the oligosaccharide chain added amino acid is further substituted with a oligosaccharide chain added amino acid, there is a tendency for blood stability to be increased and blood glucose suppressing activity to be decreased (however, it is possible to compensate for the decrease in blood glucose suppressing activity by the increase in blood stability).

In the oligosaccharide chain added antigenic GLP-1 analogue of the present invention, the site for substituting an amino acid with a oligosaccharide chain added amino acid is not particularly limited, and those skilled in the art can appropriately select a site that renders the activity to reduce antigenicity and does not reduce blood stability or blood glucose suppressing activity to lower than that of GLP-1.

In one aspect of the present invention, the site for substituting an amino acid of the antigenic GLP-1 analogue with an oligosaccharide chain added amino acid can be selected from any site in the antigenic GLP-1 analogue depending on the desired activity. For example, in the antigenic GLP-1 analogue, this is a site corresponding to one or more sites selected from positions 12, 14, 16, 20, 24, 28, 30, and 32 (= the oligosaccharide chain added amino acid is added at the amino acid at position 31) of the amino acid sequence of the GLP-1 peptide set forth in SEQ ID NO: 1, preferably a site corresponding to one or more sites selected from positions 12, 20, 24, 28, and 30, in particular a site corresponding toone or more sites selected from positions 24 and 30.

"The position corresponding to position X of the amino acid sequence of the GLP-1 peptide set forth in SEQ ID NO: 1 in the antigenic GLP-1 analogue" means the position corresponding to position X of the amino acid sequence of the GLP-1 peptide set forth in SEQ ID NO: 1 in the amino acid sequence of various the antigenic GLP-1 analogues, and said position can be easily determined by those skilled in the art based on each of the surrounding amino acid sequences etc.

For example, the amino acid sequences of antigenic GLP-1 analogues Ex-4 and liraglutide and the amino acid sequence of GLP peptide set forth in SEQ ID NO: 1 correspond as follows.

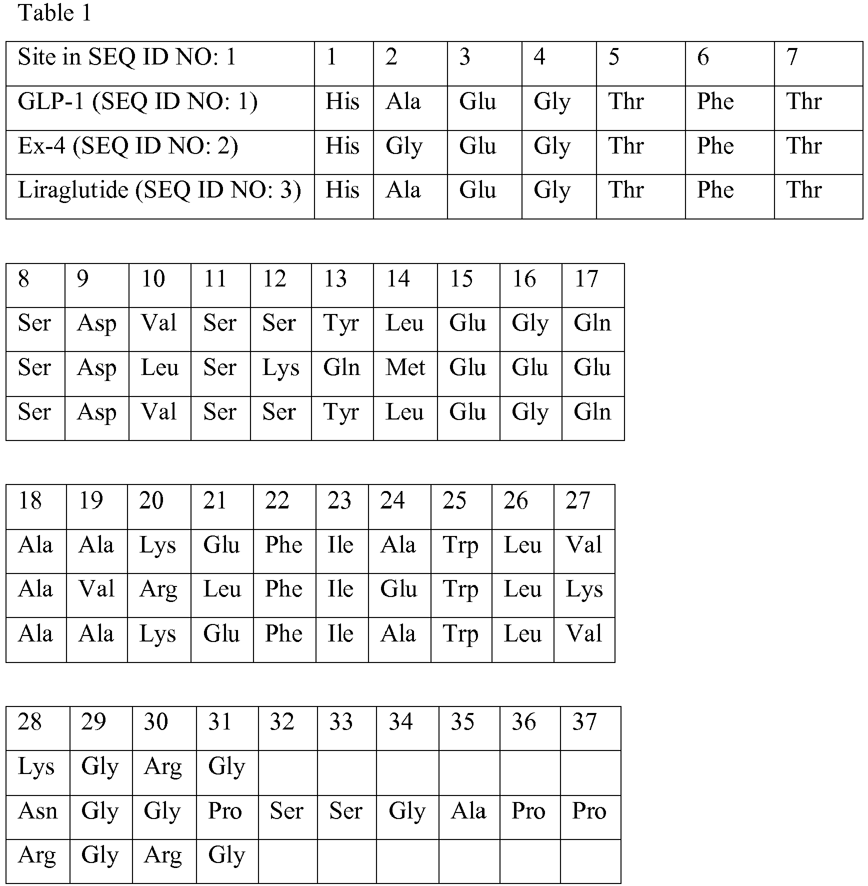

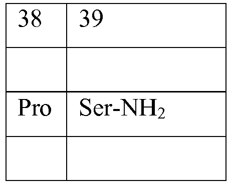

If Ex-4 or liraglutide do not have additions, substitutions, or deletions in their amino acid sequences, the vertically aligned amino acids in the above table are the amino acids at the "corresponding position." If there are additions, substitutions, or deletions, those skilled in the art can determine the "corresponding position" based on each of the surrounding amino acid sequences.

In one aspect of the present invention, the site for substituting an amino acid with a oligosaccharide chain added amino acid, in terms of reducing the antigenicity of the oligosaccharide chain added antigenic GLP-1 analogue, is, for example, preferably near the antigen recognition site in the antigenic GLP-1 analogue. Moreover, for example, it is effective to add an oligosaccharide chain to the site corresponding to position 30 of the GLP-1 peptide set forth in SEQ ID NO: 1.

In one aspect of the present invention, the site for substituting an amino acid with a oligosaccharide chain added amino acid, in terms of the blood stability of the oligosaccharide chain added antigenic GLP-1 analogue, is, for example, a site corresponding to one or more sites selected from positions 3, 4, 5, 6, 8, 10, 12, 13, 14, 16, 18, 19, 20, 21, 22, 24, 26, 28, 30, and 32 (= the oligosaccharide chain added amino acid is added at the amino acid at position 31) of the GLP-1 peptide set forth in SEQ ID NO: 1, preferably one or more sites selected from positions 3, 4, 5, 6, 8, and 22, and particularly preferably a site corresponding to one or more sites selected from 3, 4, 5, and 6. In particular, substitution of an amino acid at a site close to the N-terminal of GLP-1 is also preferred. In particular, examples of sites for substituting an amino acid with an oligosaccharide chain added amino acid can include, e.g., sites corresponding to each of positions 12 and 30, positions 20 and 28, positions 16 and 24, positions 16 and 30, and positions 24 and 30 etc.

In one aspect of the present invention, the site for substituting an amino acid with a oligosaccharide chain added amino acid, in terms of the blood glucose suppressing action of the oligosaccharide chain added antigenic GLP-1 analogue, is, for example, a site corresponding to one or more sites selected from positions 12, 14, 16, 20, 24, 28, 30, and 32 (= the oligosaccharide chain added amino acid is added at the amino acid at position 31) of the GLP-1 peptide set forth in SEQ ID NO: 1, preferably a site corresponding to one or more sites selected from positions 12, 20, 24, 28, and 30, and in particular sites corresponding to one or more sites selected from positions 24 and 30. Examples of sites for substituting two or more amino acids with oligosaccharide chain added amino acids can include, e.g., sites corresponding to each of positions 12 and 30, positions 20 and 28, positions 16 and 24, positions 16 and 30, and positions 24 and 30 etc.

In one aspect of the present invention, the site for substituting an amino acid with a oligosaccharide chain added amino acid, in terms of cAMP synthesis capability among the GLP-1 activities of the oligosaccharide chain added antigenic GLP-1 analogue, is, for example, a site corresponding to one or more sites selected from positions 16, 20, 21, 24, 28, 30, and 32 (= the oligosaccharide chain added amino acid is added at the amino acid at position 31) of the GLP-1 peptide set forth in SEQ ID NO: 1, and preferably a site corresponding to one or more sites selected from positions 16, 20, 24, 28, 30, and 32.

In one aspect of the present invention, the site for substituting an amino acid with an oligosaccharide chain added amino acid is one or more sites selected from sites other than positions 2, 3, and 6 of GLP-1 consisting of the amino acid sequence set forth in SEQ ID NO: 1.
In one aspect of the present invention, the site for substituting an amino acid with a oligosaccharide chain added amino acid is one or more sites selected from sites other than positions 1, 4, 7, 9, 13, 15, and 23 of GLP-1, and in particular one or more sites selected from sites other than positions 1, 4, and 9.

In one aspect of the present invention, the site for substituting an amino acid with an oligosaccharide chain added amino acid can also be determined from the bindig site of GLP-1 to GLP-1 receptor.

In one aspect of the present invention, when two or more amino acids are substituted with oligosaccharide chain added amino acids, any combination of the above can be employed for the sites for substituting amino acids with oligosaccharide chain added amino acids, althogh it will not be limited thereto. For example, a combination of one site being selected from the above preferred sites, and the other site being selected from any site of the antigenic GLP-1 analogue; a combination of one site being selected from the above preferred sites, and the other site being selected from any site of one or more amino acids further added to the C-terminal of the antigenic GLP-1 analogue etc. are also included in one preferred aspect of the present invention.

In one aspect of the present invention, a site where deletion, substitution, or addition of an amino acid other than the oligosaccharide chain added amino acid occurs is preferably one or more sites selected from sites other than positions 1, 4, 7, 9, 13, 15, 22, and 23 of GLP-1 consisting of the amino acid sequence set forth in SEQ ID NO: 1, e.g. one or more sites selected from sites other than positions 1, 4, 9, and 22 (Structure-Activity Studies of Glucagon-like Peptide-1, THE JOURNAL OF BIOLOGICAL CHEMISTRY Vol.269, No.9, Issue of March 4, pp. 6276-6278.1994).

The oligosaccharide chain added antigenic GLP-1 analogue of the present invention can include e.g. those having an oligosaccharide chain added to exendin-4 having the following amino acid sequence: 
H-His-Gly-Glu-Gly-Thr-Phe-Thr-Ser-Asp-Leu-Ser-Lys-Gln-Met-Glu-Glu-Glu-Ala-Val-Arg-
Leu-Phe-Ile-Glu-Trp-Leu-Lys-Asn-Gly-Gly-Pro-Ser-Ser-Gly-Ala-Pro-Pro-Pro-Ser-NH2 (SEQ
ID NO: 2).
 Oligosaccharide chain added exendin-4 is represented by e.g. the following general formula (1): 
General formula (1)
H-His-Gly-Glu-Gly-Thr-Phe-Thr-Ser-Asp-Leu-Ser-Xaa12-Gln-Xaa14-Glu-Xaa16-Glu-Ala-Val-
Xaa20-Leu-Phe-Ile-Xaa24-Trp-Leu-Lys-Xaa28-Gly-Xaa30-Pro-Ser-Ser-Gly-Ala-Pro-Pro-Pro-
Ser-NH2
[wherein Xaa₁₂ indicates Lys, oligosaccharide chain added Cys, or oligosaccharide chain added Asn;
Xaa₁₄ indicates Met, oligosaccharide chain added Cys, or oligosaccharide chain added Asn;
Xaa₁₆ indicates Glu, oligosaccharide chain added Cys, or oligosaccharide chain added Asn;
Xaa₂₀ indicates Arg, oligosaccharide chain added Cys, or oligosaccharide chain added Asn;
Xaa₂₄ indicates Glu, oligosaccharide chain added Cys, or oligosaccharide chain added Asn;
Xaa₂₈ indicates Asn, oligosaccharide chain added Cys, or oligosaccharide chain added Asn;
Xaa₃₀ indicates Gly, oligosaccharide chain added Cys, or oligosaccharide chain added Asn; and
at least one of Xaa₁₂, Xaa₁₄, Xaa₁₆, Xaa₂₀, Xaa₂₄, Xaa₂₈, and Xaa₃₀ is oligosaccharide chain added Cys or oligosaccharide chain added Asn.] (SEQ ID NO: 4)
Among these, it is preferred that Xaa₂₄ is oligosaccharide chain added Cys.

Meanwhile, for a peptide in which the C-terminal is originally amidated such as exendin-4, when synthesizing an oligosaccharide chain added amino acid having an oligosaccharide chain added to the amino acid of the C-terminal, the C-terminal is sometimes not amidated.

The oligosaccharide chain added antigenic GLP-1 analogue of the present invention can include e.g. those having an oligosaccharide chain added to liraglutide having the following amino acid sequence. 
His-Ala-Glu-Gly-Thr-Phe-Thr-Ser-Asp-Val-Ser-Ser-Tyr-Leu-Glu-Gly-Gln-Ala-Ala-Lys20-
Glu-Phe-Ile-Ala-Trp-Leu-Val-Arg-Gly-Arg-Gly (SEQ ID NO: 3)
 As shown in the following formula, a palmitoyl group is bound to Lys₂₀ via glutamic acid. 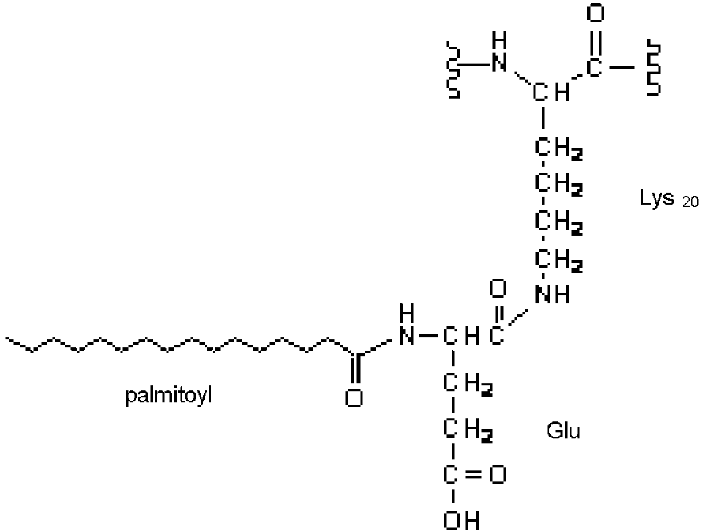 Oligosaccharide chain added liraglutide is represented by e.g. the following general formula (2): General formula (2) 
His-Ala-Glu-Gly-Thr-Phe-Thr-Ser-Asp-Val-Ser-Xaa12-Tyr-Xaa14-Glu-Xaa16-Gln-Ala-Ala-
Lys20-Glu-Phe-Ile-Xaa24-Trp-Leu-Val-Xaa28-Gly-Xaa30-Gly
 [wherein Xaa₁₂ indicates Ser, oligosaccharide chain added Cys, or oligosaccharide chain added Asn;
Xaa₁₄ indicates Leu, oligosaccharide chain added Cys, or oligosaccharide chain added Asn;
Xaa₁₆ indicates Gly, oligosaccharide chain added Cys, or oligosaccharide chain added Asn;
Xaa₂₄ indicates Ala, oligosaccharide chain added Cys, or oligosaccharide chain added Asn;
Xaa₂₈ indicates Arg, oligosaccharide chain added Cys, or oligosaccharide chain added Asn;
Xaa₃₀ indicates Arg, oligosaccharide chain added Cys, or oligosaccharide chain added Asn; and
at least one of Xaa₁₂, Xaa₁₄, Xaa₁₆, Xaa₂₄, Xaa₂₈, and Xaa₃₀ is oligosaccharide chain added Cys or oligosaccharide chain added Asn.] (SEQ ID NO: 5)
Among these, it is preferred that Xaa₂₄ and/or Xaa₃₀ are oligosaccharide chain added Cys or oligosaccharide chain added Asn, and in particular, it is preferred that Xaa₃₀ is oligosaccharide chain added Cys.

Moreover, the oligosaccharide chain added antigenic GLP-1 analogue of the present invention can include e.g. those having an oligosaccharide chain added to BIM51077 having the following amino acid sequence: 
His-R2-Glu-Gly-Thr-Phe-Thr-Ser-Asp-Val-Ser-Ser-Tyr-Leu-Glu-Gly-Gln-Ala-Ala-Lys-Glu-
Phe-Ile-Ala-Trp-Leu-Val-Lys-R2-Arg-NH2
 [wherein R2 indicates α-methylalanine (aminoisobutanoic acid, also referred to as Aib).] (SEQ ID NO: 18)
Oligosaccharide chain added BIM51077 is represented by e.g. the following general formula (3): 
General formula (3)
His-R2-Glu-Gly-Thr-Phe-Thr-Ser-Asp-Val-Ser-Xaa18-Tyr-Xaa20-Glu-Xaa22-Gln-Ala-Ala-
Xaa26-Glu-Phe-Ile-Xaa30-Trp-Leu-Val-Xaa34-R2-Xaa36-NH2
 [wherein R2 indicates α-methylalanine;
Xaa₁₈ indicates Ser, oligosaccharide chain added Cys, or oligosaccharide chain added Asn;
Xaa₂₀ indicates Leu, oligosaccharide chain added Cys, or oligosaccharide chain added Asn;
Xaa₂₂ indicates Gly, oligosaccharide chain added Cys, or oligosaccharide chain added Asn;
Xaa₂₆ indicates Lys, oligosaccharide chain added Cys, oligosaccharide chain added Asn, or oligosaccharide chain added Lys;
Xaa₃₀ indicates Ala, oligosaccharide chain added Cys, or oligosaccharide chain added Asn.
Xaa₃₄ indicates Lys, oligosaccharide chain added Cys, oligosaccharide chain added Asn, or oligosaccharide chain added Lys;
Xaa₃₆ indicates Arg, oligosaccharide chain added Cys, or oligosaccharide chain added Asn; and
at least one of Xaa₁₈, Xaa₂₀, Xaa₂₂, Xaa₂₆, Xaa₃₀, Xaa₃₄, and Xaa₃₆ is oligosaccharide chain added Cys or oligosaccharide chain added Asn.] (SEQ ID NO: 19)

An "oligosaccharide chain" herein refers to a compound generated by one or more unit sugars (monosaccharide and/or derivatives thereof) in line. When two or more unit sugars are in line, each unit sugar is bound to each other by dehydration condensation by a glycoside bond. Such oligosaccharide chains include a broad range including, but are not limited to, e.g., monosaccharide and polysaccharides (glucose, galactose, mannose, fucose, xylose, N-acetylglucosamine, N-acetylgalactosamine, sialic acid, and complexes and derivatives thereof) contained in vivo, as well as oligosaccharide chains degradated or derived from complex biomolecules such as degradated polysaccharides, glycoproteins, proteoglycans, glycosaminoglycans, and glycolipids etc. The oligosaccharide chain may be linear or branched.

"Oligosaccharide chains" herein also include oligosaccharide chain derivatives, and oligosaccharide chain derivatives include, but are not limited to, oligosaccharide chains which are: e.g., a sugar in which the sugar constituting the oligosaccharide chain have a carboxyl group (e.g., aldonic acid having the C-1 position oxidized to carboxylic acid (e.g., D-glucose oxidized to D-gluconic acid), uronic acid having the terminal C atom turned into carboxylic acid (D-glucose oxidized to D-glucuronic acid)), a sugar having an amino group or amino group derivative (e.g. acetylated amino group) (e.g., N-acetyl-D-glucosamine and N-acetyl-D-galactosamine), a sugar having both amino and carboxyl groups (e.g., N-acetylneuraminic acid (sialic acid) and N-acetylmuramic acid), a deoxylated sugar (e.g. 2-deoxy-D-ribose), a sulfated sugar comprising a sulfate group, and a phosphorylated sugar comprising a phosphate group.

In the present invention, the preferred oligosaccharide chain is a oligosaccharide chain which when added to an antigenic GLP-1 analogue (when substituted with an amino acid of the antigenic GLP-1 analogue in the form of a oligosaccharide chain added amino acid), reduces the antigenicity of the antigenic GLP-1 analogue, preferably increases the blood stability, and more preferably does not diminish the blood glucose suppressing activity. However, blood stability and/or blood glucose suppressing activity may be equal to the antigenic GLP-1 analogue as long as the antigenicity is reduced.

The oligosaccharide chain in the oligosaccharide chain added antigenic GLP-1 analogue of the present invention is not particularly limited, and it may be an oligosaccharide chain that exists in vivo as a glycoconjugate (glycopeptide (or glycoprotein), proteoglycan, and glycolipid etc.), or may be an oligosaccharide chain that does not exist in vivo as a glycoconjugate.

An oligosaccharide chain that exists in vivo as a complex carbohydrate is preferred in terms of the oligosaccharide chain added antigenic GLP-1 analogue of the present invention being administered to organisms. Said oligosaccharide chains include N-linked oligosaccharide chains and O-linked oligosaccharide chains etc., which are oligosaccharide chains that are bound in vivo to a peptide (or protein) as a glycopeptide (or glycoprotein). N-linked oligosaccharide chains are preferably used. N-linked oligosaccharide chains can include e.g. high-mannose form, complex form, and hybrid form, particularly preferably complex form.

Preferred complex oligosaccharide chains used in the present invention include e.g. oligosaccharide chains etc. represented by the following general formula: 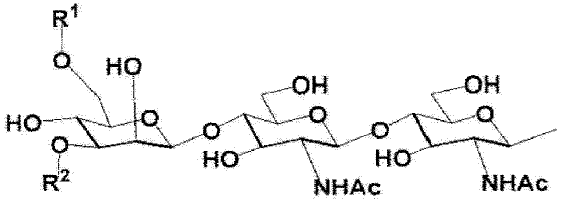 [wherein R¹ and R² are the same or different, and indicate: 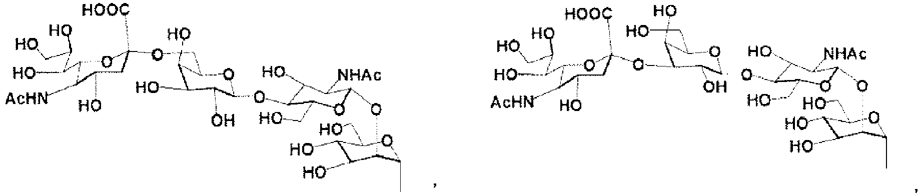 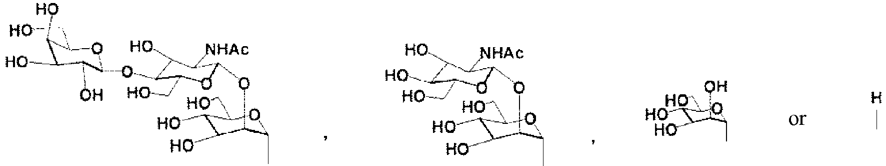 and Ac indicates an acetyl group.]

Further, in the oligosaccharide chain added antigenic GLP-1 analogue of the present invention, if the oligosaccharide chain is one that exists in vivo as a glycoconjugate, it may be bound to the antigenic GLP-1 analogue by a method other than O-linkage and N-linkage. For example, as described above, one where oligosaccharide chain is bound to Cys etc. via a linker is also included in the oligosaccharide chain added antigenic GLP-1 analogue of the present invention.

In one aspect of the present invention, the oligosaccharide chain in the oligosaccharide chain added antigenic GLP-1 analogue of the present invention is preferably an oligosaccharide chain consisting of 4 or more, e.g. 5 or more, 7 or more, in particular 9 or more, and 11 or more sugars.

In one preferred aspect of the present invention, the oligosaccharide chain in the oligosaccharide chain added antigenic GLP-1 analogue of the present invention is an oligosaccharide chain consisting of 5 to 11, 9 to 11, 9, 11 sugars.

In one preferred aspect of the present invention, the oligosaccharide chain in the oligosaccharide chain added antigenic GLP-1 analogue of the present invention is a double-stranded complex oligosaccharide chain. A complex oligosaccharide chain is characterized in that it comprises two or more kinds of monosaccharide, and has a basic structure shown below and a lactosamine structure shown by Galβ 1-4GlcNAc. 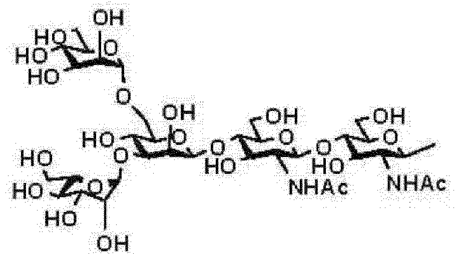 A double-stranded complex oligosaccharide chain refers to one where single-stranded oligosaccharide chains consisting of 0 to 3 sugars are bound to each of the two mannoses at the termini of the basic structure. Preferred double-stranded complex oligosaccharide chains are, e.g., as shown below, a disialooligosaccharide chain: 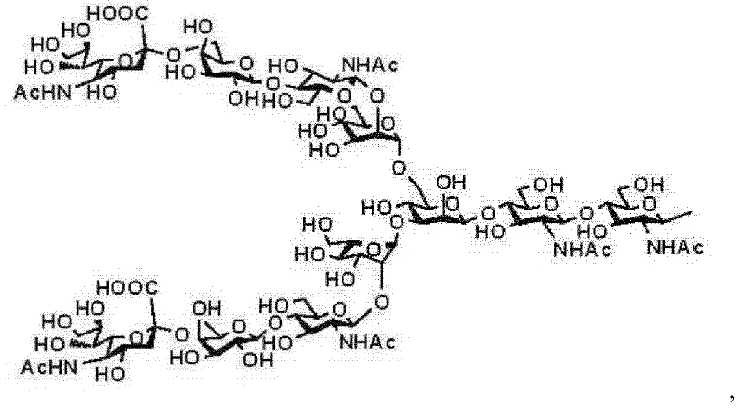 a monosialooligosaccharide chain: 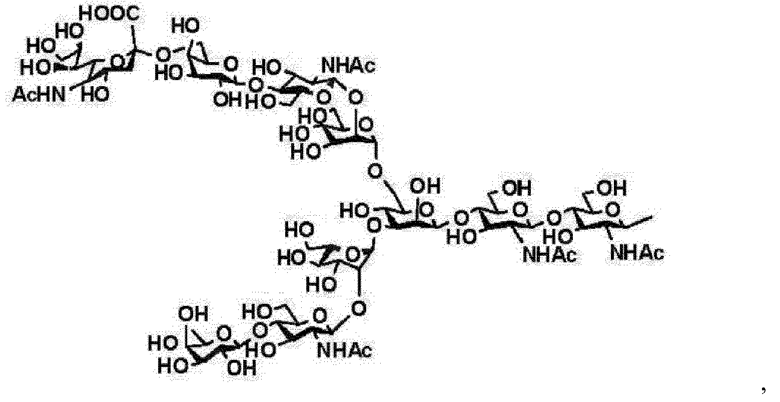 an asialooligosaccharide chain: 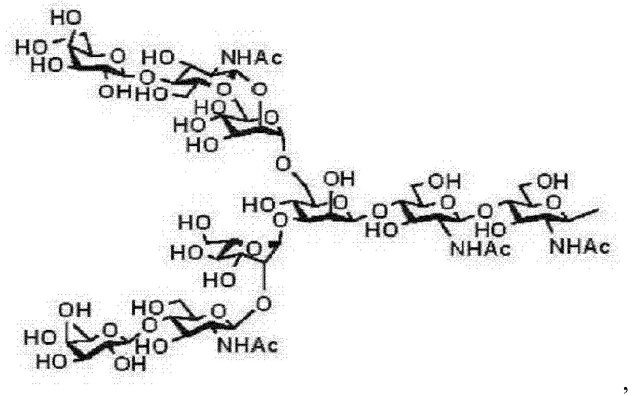 and a diGlucNAc oligosaccharide chain: 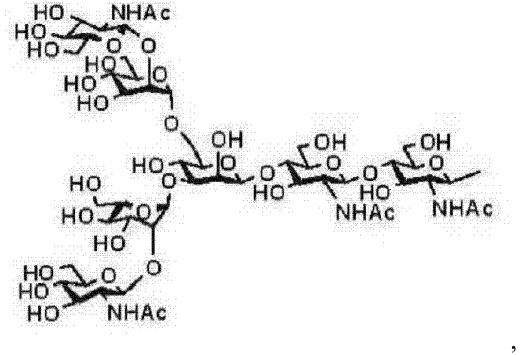 and dimannose oligosaccharide chain: 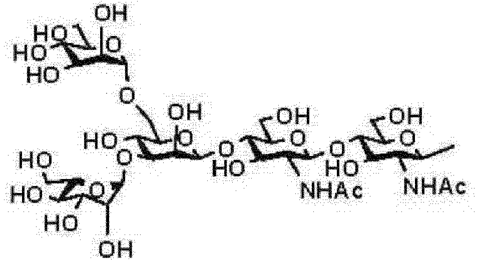 etc., and more preferably a disialooligosaccharide chain, a monosialooligosaccharide chain, or an asialooligosaccharide chain.
Moreover, "disialooligosaccharide chains," "monosialooligosaccharide chains," "asialooligosaccharide chains," "diGlucNAc oligosaccharide chains," and "dimannose oligosaccharide chains" in the present invention also include, in addition to those shown in the above chemical formulae, those bound by a binding pattern different from the binding pattern of the examples shown in the chemical formulae, and these oligosaccharide chains are also preferably employed as oligosaccharide chains of the present invention. These oligosaccharide chains include, e.g., those where sialic acid and galactose are bound by a (α2 -> 3) bond in the disialooligosaccharide chain and asialooligosaccharide chain etc.

Further, the high-mannose oligosaccharide chain employed in the present invention is an oligosaccharide chain where two or more mannoses are further bound to the basic structure of the above-described complex oligosaccharide chain. Since high-mannose oligosaccharide chain is bulky, blood stability may become higher by binding a high-mannose oligosaccharide chain to a peptide. An oligosaccharide chain comprising 5 to 9 mannoses as in a mammalian high-mannose oligosaccharide chain is preferred, but it may be an oligosaccharide chain comprising more mannoses as in a high-mannose oligosaccharide chain of yeasts. High-mannose oligosaccharide chains preferebly used in the present invention can include, e.g.:
a high-mannose-5 (M-5): 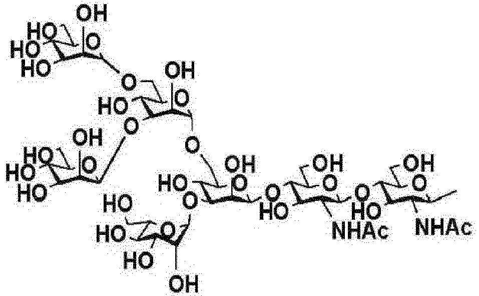 and a high-mannose-9 (M-9): 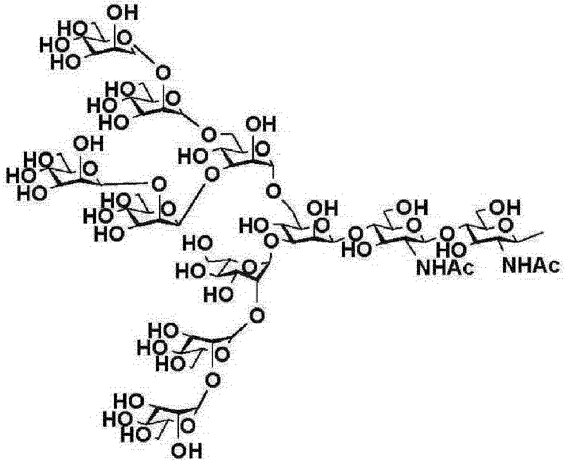
etc.

In the present invention, the preferred oligosaccharide chain can include, e.g., a oligosaccharide chain having the same structure (a oligosaccharide chain with the same type of constituent sugars and binding patterns thereof) as a oligosaccharide chain that exists in the human body as a glycoprotein bound to a protein (e.g., the oligosaccharide chain described in "FEBS LETTERS Vol.50, No.3, Feb.1975"), or a oligosaccharide chain having one or more sugar deleted from the non-reducing terminal thereof, which are the oligosaccharide chains shown by the following Tables 2 to 5.

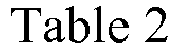

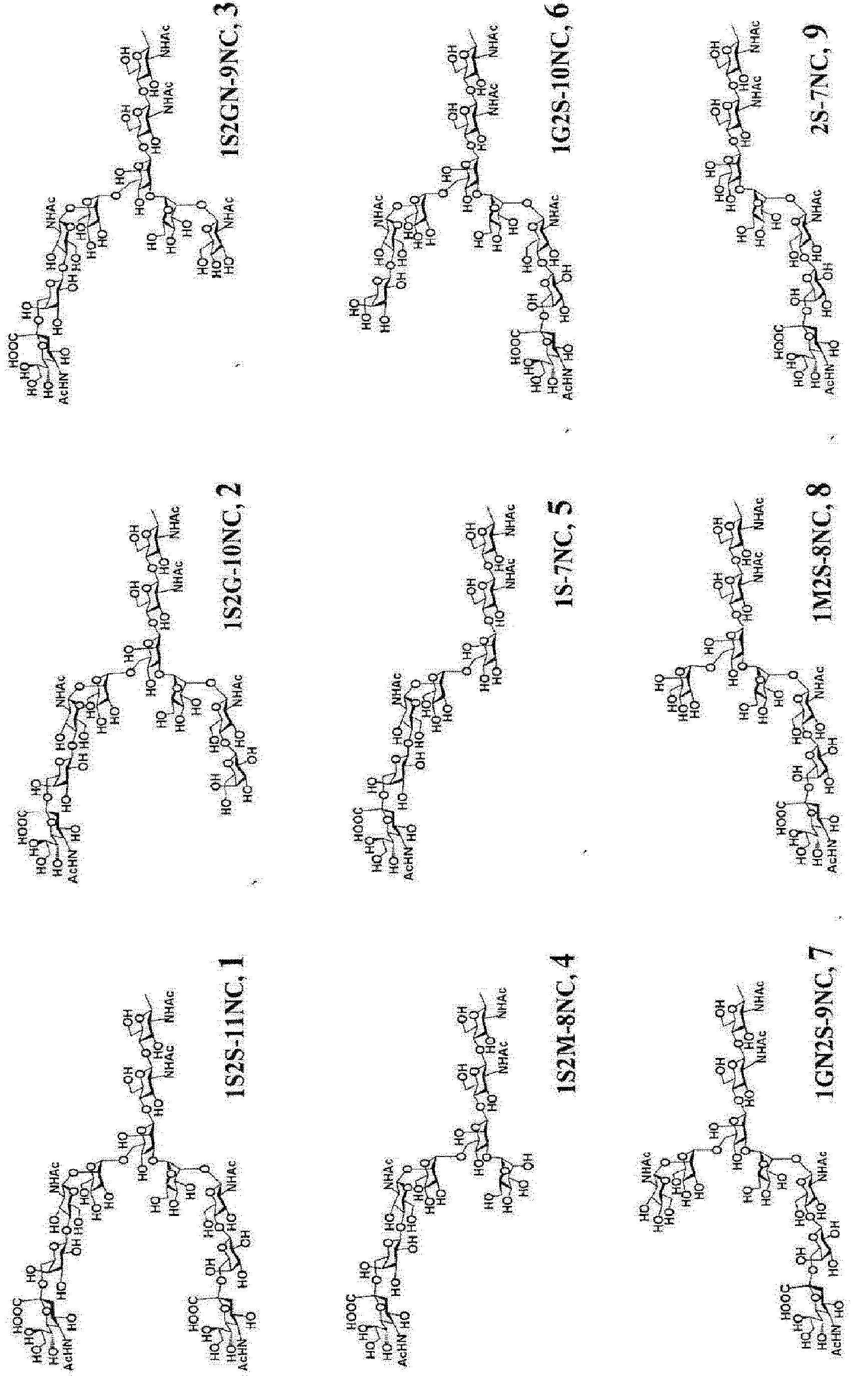

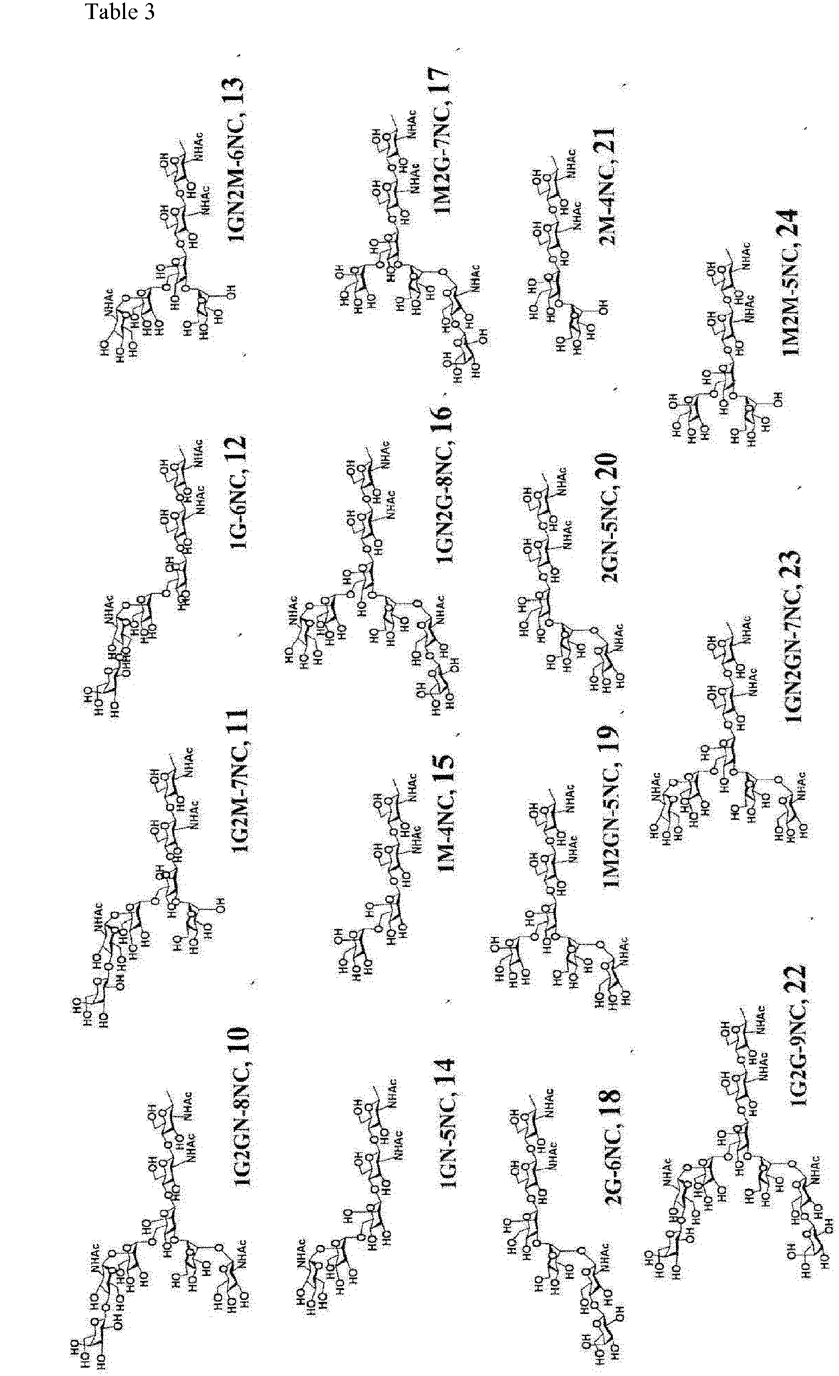

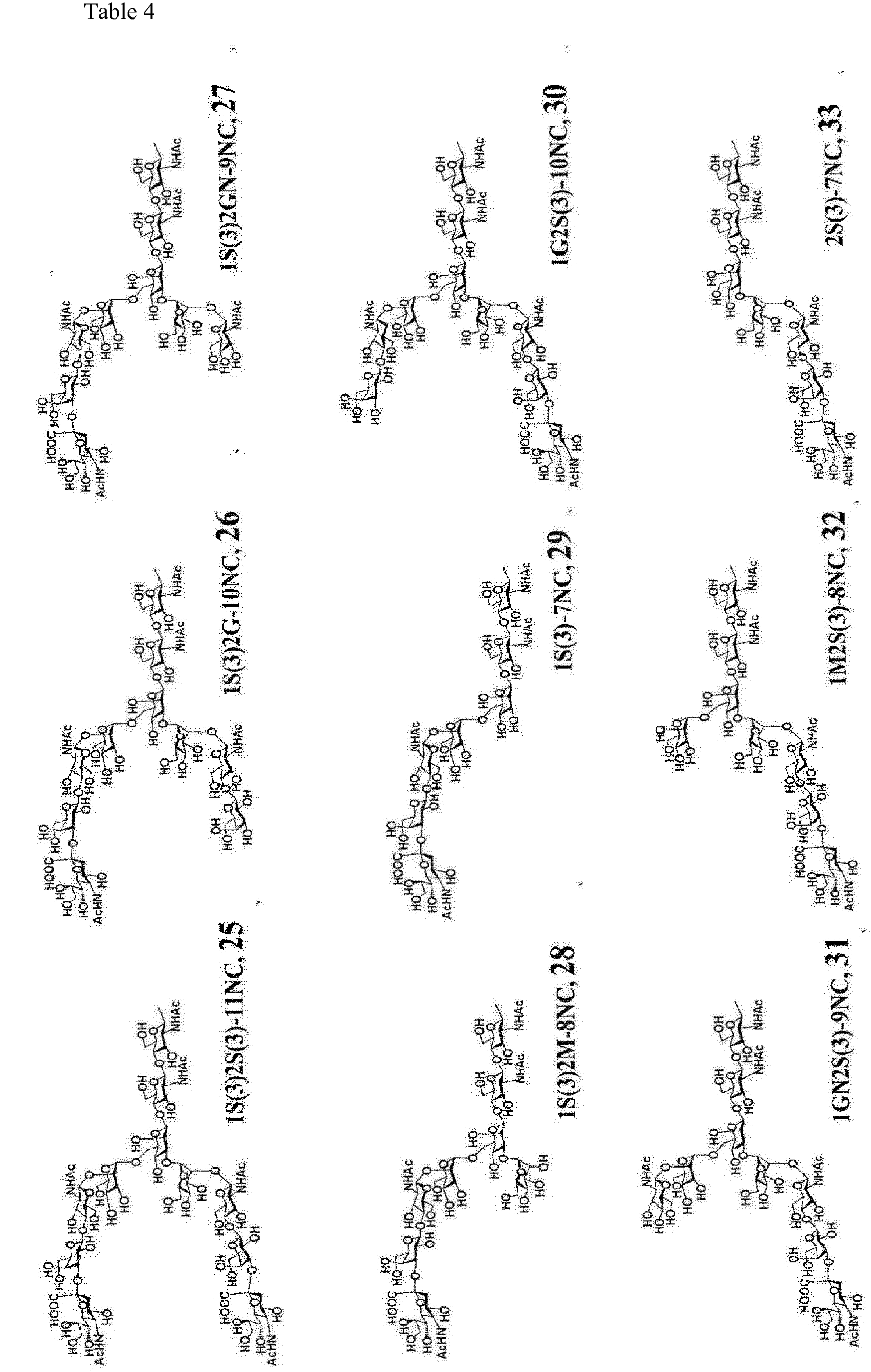

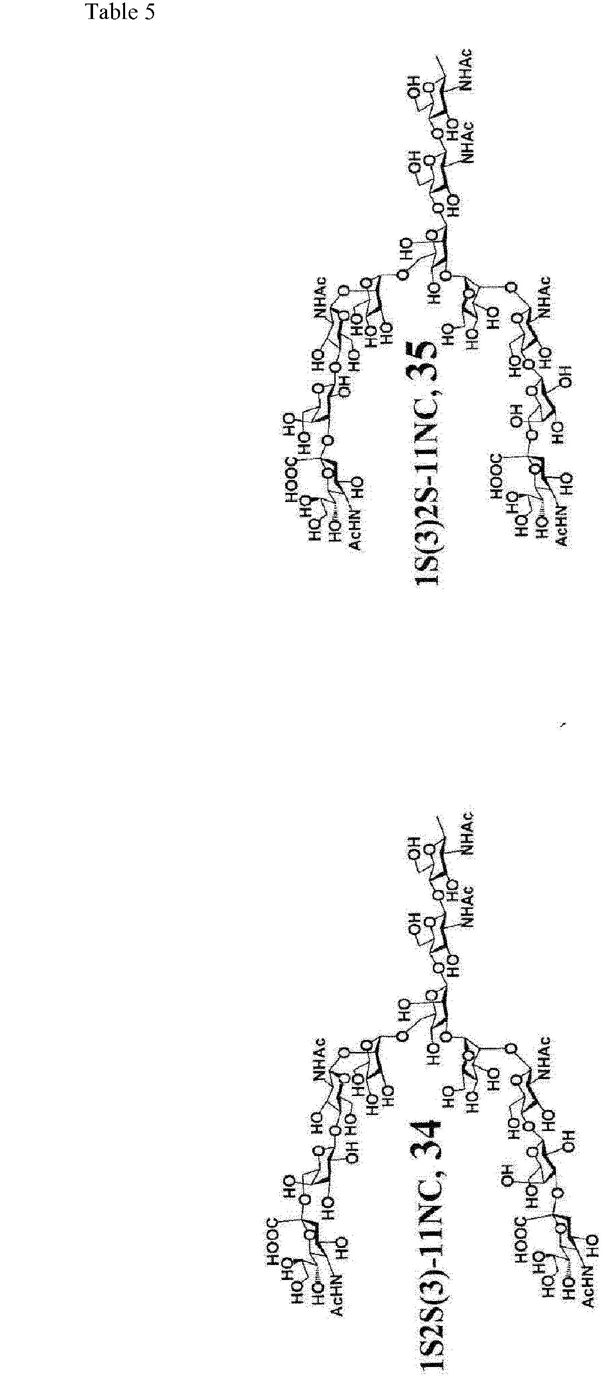

In one preferred aspect of the present invention, the oligosaccharide chain structures of the glycopeptide of the present invention are homogeneous. As used herein, oligosaccharide chain structures in the glycopeptide are homogeneous, means that the glycosylation sites in the peptide, as well as the type, binding order, and binding pattern of each sugar constituting the oligosaccharide chain are the same when compared between glycopeptides, and means that at least 90% or more, preferably 95% or more, and more preferably 99% or more oligosaccharide chain structures are homogeneous. A glycopeptide having homogeneous oligosaccharide chains are constant in quality, and is preferred in particular for fields of pharmaceuticals manufacture or assays. The proportion of homogeneous oligosaccharide chains can be measured by for example methods employing HPLC, capillary electrophoresis, NMR, and mass spectrometry etc.

In the present invention, preferred oligosaccharide chain added antigenic GLP-1 analogues are, e.g., oligosaccharide chain added antigenic GLP-1 analogues manufactured in Examples 1 to 4 described below.
Specifically, they are:
(a1) a oligosaccharide chain added antigenic GLP-1 analogue wherein Gly at position 30 is substituted with a disialooligosaccharide chain added Cys in exendin-4 consisting of the amino acid sequence set forth in SEQ ID NO: 2 (Example 1) (SEQ ID NO: 6);
(a2) a oligosaccharide chain added antigenic GLP-1 analogue wherein Gly at position 30 is substituted with a high-mannose-type 5-oligosaccharide chain added Cys in exendin-4 consisting of the amino acid sequence set forth in SEQ ID NO: 2(Example 2) (SEQ ID NO: 7);
(a3) a oligosaccharide chain added antigenic GLP-1 analogue wherein Arg at position 30 is substituted with an asialooligosaccharide chain added Cys in liraglutide consisting of the amino acid sequence set forth in SEQ ID NO: 3 (Example 3) (SEQ ID NO: 8);
(a4) a oligosaccharide chain added antigenic GLP-1 analogue wherein Arg at position 30 is substituted with a disialooligosaccharide chain added Cys in liraglutide consisting of the amino acid sequence set forth in SEQ ID NO: 3 (Example 4) (SEQ ID NO: 9) (a5) a oligosaccharide chain added antigenic GLP-1 analogue wherein Lys at position 20 is substituted with a disialooligosaccharide chain added Cys in BIM-50177 consisting of the amino acid sequence set forth in SEQ ID NO: 18 (Example 5) (SEQ ID NO: 21)

### (Manufacturing Methods)

The oligosaccharide chain added antigenic GLP-1 analogue of the present invention can be manufactured by incorporating a glycosylation step into peptide synthesis methods well-known to those skilled in the art. A method that utilizes the reverse reaction of enzymes represented by trans-glutaminase can also be employed for glycosylation, but a large amount of oligosaccharide chains to be added will be necessary for this, causing problems such as purification after the final step becoming complicated, and positions for adding oligosaccharide chains and oligosaccharide chains that can be added becoming limited etc., and therefore, although this method can be employed for small-scale synthesis for assays etc., it may not be practical for large-scale manufacturing for pharmaceuticals manufacture etc.

As specific examples of simple manufacturing methods for the oligosaccharide chain added antigenic GLP-1 analogue of the present invention, wherein the manufacturing methods are stable methods for oligosaccharide chain added antigenic GLP-1 analogues having homogeneous oligosaccharide chain structures, the following are examplified: a method for manufacturing oligosaccharide chain added antigenic GLP-1 analogue by using oligosaccharide chain added Asn as the oligosaccharide chain added amino acid and appling well-known peptide synthesis methods such as solid-phase synthesis and liquid-phase synthesis (method A), and a method for manufacturing oligosaccharide chain added antigenic GLP-1 analogue by manufacturing a peptide having any amino acid of the antigenic GLP-1 analogue substituted with Cys according to a well-known peptide synthesis method, and then adding a oligosaccharide chain to Cys by chemical synthesis (method B). Further, a method for manufacturing oligosaccharide chain added antigenic GLP-1 analogue by binding oligosaccharide chain added Asn to one end of the linker, and then binding N-hydroxysuccinic acid imidyl group to the other end of the linker, and reacting the N-hydroxysuccinic acid imidyl group with the side chain amino group of Lys residue of the antigenic GLP-1 analogue (method C) is shown. Referring to these manufacturing methods, those skilled in the art will be able to manufacture various oligosaccharide chain added antigenic GLP-1 analogues, and the oligosaccharide chain added antigenic GLP-1 analogues obtained and manufacturing methods thereof are very useful especially in the pharmaceuticals manufacture field.

These methods A to C can also be employed in a combination of two. In the case of small-scale synthesis e.g. for assays, it is also possible to combine oligosaccharide chain elongation reaction by a transferase with the above methods. Method A is described in International Publication No. WO2004/005330 (US2005222382 (A1)), and method B is described in International Publication No. WO2005/010053 (US2007060543 (A1)), the disclosures of which are herein incorporated by reference in their entirety. The manufacture of oligosaccharide chains having homogeneous oligosaccharide chain structures employed in methods A to C are also described in WO 03/008431 (US2004181054 (A1)), WO 2004/058984 (US2006228784 (A1)), WO 2004/058824 (US2006009421 (A1)), WO 2004/070046 (US2006205039 (A1)), and WO 2007/011055 etc., the disclosures of which are herein incorporated by reference in their entirety.

### Method for Manufacturing Oligosaccharide chain added Antigenic GLP-1 Analogue (Method A)

Oligosaccharide chain added antigenic GLP-1 analogue can be manufactured by e.g. solid-phase synthesis employing oligosaccharide chain added asparagine as summarized below.
(1) The hydroxyl group of a resin having a hydroxyl group and the carboxyl group of an amino acid having the amino group nitrogen protected with a lipophilic protective group are subjected to esterification reaction. In this case, since the amino group nitrogen of the amino acid is protected with a lipophilic protective group, self-condensation between amino acids is prevented, and the hydroxyl group of the resin and the carboxyl group of the amino acid react and esterification occurs.
(2) The lipophilic protective group of the ester obtained is detached to form a free amino group.
(3) This free amino group and the carboxyl group of any amino acid having the amino group nitrogen protected with a lipophilic protective group are subjected to amidation reaction.
(4) The above lipophilic protective group is detached to form a free amino group.
(5) By repeating the above steps (3) and (4) once or more times, a peptide of any number of any amino acids linked together, having the resin bound on one end and having a free amino group on the other end is obtained.
(6) Finally, a peptide having the desired amino acid sequence can be obtained by cleaving the resin with an acid.
In step (1) here, if a oligosaccharide chain added asparagine having the amino group nitrogen protected with a lipophilic protective group is employed instead of an amino acid having the amino group nitrogen protected with a lipophilic protective group, and the carboxyl group of such asparagine portion and the hydroxyl group of the resin are reacted, a peptide having oligosaccharide chain added asparagine at the C-terminal can be obtained.
Moreover, after step (2), or after repeating steps (3) and (4) for any number of times that is once or more, if a oligosaccharide chain added asparagine having the amino group nitrogen protected with a lipophilic protective group is employed in step (3) instead of an amino acid having the amino group nitrogen protected with a lipophilic protective group, the oligosaccharide chain can be added at any place.
Further, in any of steps (1) and (3), if a oligosaccharide chain added asparagine having the amino group nitrogen protected with a lipophilic protective group is employed instead of an amino acid having the amino group nitrogen protected with a lipophilic protective group twice or more times, a peptide having oligosaccharide chains added at any two or more places can be obtained.
After binding the oligosaccharide chain added amino acid, the lipophilic protective group is detached to form a free amino group, and if step (6) is performed immediately thereafter, a peptide having an oligosaccharide chain added asparagine at the N-terminal can be obtained.

The resin having a hydroxyl group may typically be a resin having a hydroxyl group used in solid-phase synthesis, and e.g. Amino-PEGA resin (Merck & Co., Inc.), Wang resin (Merck & Co., Inc.), and HMPA-PEGA resin (Merck & Co., Inc.) etc. can be used.
In addition, when the C-terminal is to be amidated as in Ex-4, it is preferable to use Rink-Amido-PEGA resin (Merck & Co., Inc.). The C-terminal amino acid of the peptide can be amidated by cleaving this resin and the peptide with an acid.

Any amino acid can be employed as the amino acid, and can include e.g. the natural amino acids serine (Ser), asparagine (Asn), valine (Val), leucine (Leu), isoleucine (Ile), alanine (Ala), tyrosine (Tyr), glycine (Gly), lysine (Lys), arginine (Arg), histidine (His), aspartic acid (Asp), glutamic acid (Glu), glutamine (Gln), threonine (Thr), cystein (Cys), methionine (Met), phenylalanine (Phe), tryptophan (Trp), and proline (Pro).

Lipophilic protective groups can include, e.g., carbonate-based or amido-based protective groups etc. such as 9-fluorenylmethoxycarbonyl (Fmoc) group, t-butyloxycarbonyl (Boc) group, benzyl group, allyl group, allyloxycarbonyl group, and acetyl group etc. For introducing a lipophilic protective group to an amino acid, for example for introducing an Fmoc group, introduction can be carried out by adding 9-fluorenylmethyl-N-succinimidyl carbonate and sodium bicarbonate for reaction. The reaction may be performed at 0 to 50°C, preferably at room temperature for about 1 to 5 hours.

The amino acid protected with a lipophilic protective group used may also be those commercially available. Examples can include Fmoc-Ser, Fmoc-Asn, Fmoc-Val, Fmoc-Leu, Fmoc-Ile, Fmoc-Ala, Fmoc-Tyr, Fmoc-Gly, Fmoc-Lys, Fmoc-Arg, Fmoc-His, Fmoc-Asp, Fmoc-Glu, Fmoc-Gln, Fmoc-Thr, Fmoc-Cys, Fmoc-Met, Fmoc-Phe, Fmoc-Trp, and Fmoc-Pro etc.

Well-known dehydration condensation agents, e.g., 1-mesitylenesulfonyl-3-nitro-1,2,4-triazole (MSNT), dicyclohexylcarbodiimide (DCC), and diisopropylcarbodiimide (DIPCDI) etc. can be employed as the esterification catalyst. The proportion used for the amino acid and the dehydration condensation agent is 1 part by weight of the former to typically 1 to 10 parts by weight, preferably 2 to 5 parts by weight of the latter.

Esterification reaction is preferably performed by for example placing the resin in a solid-phase column, washing this resin with a solvent, and then adding the amino acid solution. Wash solvents can include e.g. dimethyl formamide (DMF), 2-propanol, and methylene chloride etc. Solvents for dissolving the amino acid can include e.g. dimethyl sulfoxide (DMSO), DMF, and methylene chloride etc. The esterification reaction may be performed at 0 to 50°C, preferably at room temperature for about 10 minutes to 30 hours, preferably for about 15 minutes to 24 hours.

It is also preferable here to acetylate the unreacted hydroxyl groups on the solid-phase with acetic anhydride etc. for capping.

Detachment of the lipophilic protective group can be performed by for example treating with a base. Bases can include e.g. piperidine and morpholine etc. This is preferably carried out in the presence of a solvent. Solvents can include e.g. DMSO, DMF, and methanol etc.

The amidation reaction between the free amino group and the carboxyl group of any amino acid having the amino group nitrogen protected with a lipophilic protective group is preferably carried out in the presence of an activating agent and a solvent.

Activating agents can include, e.g., dicyclohexylcarbodiimide (DCC), 1-ethyl-3-(3-dimetylaminopropyl)carbodiimide hydrochloride salt (WSC/HCl), diphenylphosphorylazide (DPPA), carbonyldiimidazole (CDI), diethylcyanophosphonate (DEPC), benzotriazol-1-yloxy-tris-pyrrolidinophosphonium (DIPCI), benzotriazol-1-yloxy-tris-pyrrolidinophosphonium hexafluorophosphate (PyBOP), 1-hydroxybenzotriazole (HOBt), hydroxysuccinimide (HOSu), dimetylaminopyridine (DMAP), 1-hydroxy-7-azabenzotriazole (HOAt), hydroxyphthalimide (HOPht), pentafluorophenol (Pfp-OH), 2-(1H-benzotriazol-1-yl)-1,1,3,3-tetramethyluronium hexafluorophosphate (HBTU), O-(7-azabenzotriazol-1-yl)-1,1,3,3-tetramethyluronium hexafluorophosphonate (HATU), O-benzotriazol-1-yl-1,1,3,3-tetramethyluronium tetrafluoroborate (TBTU), and 3,4-dihydro-3-hydroxy-4-oxo-1,2,3-benzotriazine (Dhbt) etc.

The amount of the activating agent used, relative to any amino acid having the amino group nitrogen protected with a lipophilic protective group, is preferably 1 to 20 equivalents, preferably 1 to 10 equivalents, and more preferably 1 to 5 equivalents.

Solvents can include e.g. DMSO, DMF, and methylene chloride etc. The reaction may be performed at 0 to 50°C, preferably at room temperature for about 10 to 30 hours, preferably for about 15 minutes to 24 hours. Detachment of the lipophilic protective group can be performed as above.

It is preferable to use acid treatment for cleaving the peptide chain from the resin. Acids can include e.g. trifluoroacetic acid (TFA) and hydrofluoric acid (HF) etc.

In this way, an oligosaccharide chain added antigenic GLP-1 analogue having the desired position substituted with oligosaccharide chain added asparagine can be obtained.

### Method for Manufacturing Oligosaccharide chain added Antigenic GLP-1 Analogue (Method B)

The oligosaccharide chain added antigenic GLP-1 analogue can also be manufactured by a method of first synthesizing a peptide chain, and subsequently adding an oligosaccharide chain. Specifically, a peptide comprising Cys at the position to which the oligosaccharide chain is added is manufactured by methods of solid-phase synthesis, liquid-phase synthesis, synthesis by cells, and separation and extraction of those that occur in nature etc.
Next, the oligosaccharide chain is bound to the peptide by reacting the haloacetamidated complex oligosaccharide chain derivative with the peptide comprising Cys obtained above. The above reaction may typically be performed at 0 to 80°C, preferaby at 10 to 60°C, and further preferably at 15 to 35°C. The reaction time is preferably typically about 30 minutes to 5 hours. After completion of the reaction, this may be appropriately purified by a well-known method (e.g., high performance column chromatography (HPLC)).

The haloacetamidated complex oligosaccharide chain derivative is, for example, a compound where the hydroxyl group bound to the carbon at position 1 of the complex asparagine-linked oligosaccharide chain is substituted with -NH-(CO)-(CH₂)ₐ-CH₂X (wherein X is a halogen atom; and a is an integer, and is not particularly limited as long as it does not inhibit target linker function, but preferably indicates an integer between 0 and 4).

Specifically, the haloacetamidated complex oligosaccharide chain derivative and the Cys-containing peptide are reacted in phosphate buffer at room temperature. After completion of the reaction, purification by HPLC can yield an oligosaccharide chain added antigenic GLP-1 analogue substituted with oligosaccharide chain added Cys.

When oligosaccharide chain added liraglutide is manufactured as the oligosaccharide chain added antigenic GLP-1 analogue of the present invention, a step of adding a fatty acid to the Lys residue is performed. Such step can be performed by for example reacting Pal-Glu(OBu)-OSu with a peptide comprising Lys. The step of adding a fatty acid may be performed before or after the glycosylation step.

### Method for Manufacturing Oligosaccharide chain added GLP-1 Peptide (Method C)

This method in useful when the antigenic GLP-1 analogue to which an oligosaccharide chain is added comprises a Lys residue.
First, a peptide comprising Lys is manufactured by methods of solid-phase synthesis, liquid-phase synthesis, synthesis by cells, and separation and extraction of those that occur in nature etc.
Next, glutaric acid is bound to the oligosaccharide chain added amino acid. For example, the oligosaccharide chain added amino acid is dissolved in DMSO solution, a solution of glutaric acid-EDC mixure in DMSO is added to this, and stirred at room temperature for one day. After the reaction mixture is appropriately diluted, it is fractionated with molecular weight exclusion gel chromatography etc., and an oligosaccharide chain added amino acid having glutaric acid bound to the α-amino group can be obtained.
Then, to the solution of glutaric acid-bound oligosaccharide chain added amino acid in DMSO, a solution of N-hydroxysuccinic acid imide in DMSO and a solution of EDC in DMSO are added, and after stirring at room temperature for 6 hours, EDC is inactivated, and an N-hydroxysuccinic acid imidyl ester of glutaric acid-bound oligosaccharide chain added amino acid is synthesized.
Subsequently, to a solution of antigenic GLP-1 analogue in DMSO, DIPEA and the N-hydroxysuccinic acid imidyl ester of glutaric acid-bound oligosaccharide chain added amino acid are added, and after stirring at room temperature for 2 hours, aqueous glycine solution is added to terminate the reaction and appropriatly purified, and the oligosaccharide chain added amino acid can be bound to the Lys residue of the antigenic GLP-1 analogue via a glutaric acid linker.
By substituting the amino acid at the desired site of the antigenic GLP-1 analogue with Lys, or substituting the Lys residue comprised in the wild-type antigenic GLP-1 with another amino acid, it will be possible to obtain an oligosaccharide chain added antigenic GLP-1 analogue having an oligosaccharide chain added amino acid bound at the desired site. According to method C, when glycosylation is to Lys comprised in the wild-type antigenic GLP-1 analogue, an oligosaccharide chain added antigenic GLP-1 analogue having the same peptide structure as the wild-type can also be obtained.

### (Activity)

The oligosaccharide chain added antigenic GLP-1 analogue of the present invention has GLP-1 activity. The oligosaccharide chain added antigenic GLP-1 analogue of the present invention preferably has GLP-1 activity equal to or higher than that of the natural form GLP-1, and more preferably has GLP-1 activity equal to or higher compared to an antigenic GLP-1 analogue without any oligosaccharide chains added thereto (hereinafter sometimes referred to as "non-oligosaccharide chain added antigenic GLP-1 analogue.")

"GLP-1 activity" herein refers to a part or all of physiological activities well-known for GLP-1. In addition to blood glucose suppressing action, GLP-1 is known to have, e.g., islet actions such as insulin excretion accompanying cAMP synthesis induction, islet protection (apoptosis suppression), and islet proliferation, and extrapancreatic actions such as appetite suppression, gastrointestinal motility suppression, calcitonin excretion promotion, and cardioprotection action upon ischemia etc. Accordingly, GLP-1 activity refers to all or a part of physiological activities related to these actions, and can each be measured using means well-known to those skilled in the art.

For example, among GLP-1 activities, blood glucose suppressing activity can be measured using the measurement of blood glucose lowering action in diabetic mice (db/db mice), or measurement of blood glucose elevation suppressive action in an oral glucose tolerance test (OGTT). "Blood glucose suppressing" herein comprises both concepts of the suppression of rise in blood glucose and the reduction of blood glucose. In particular, in the present specification, blood glucose suppressing action in db/db mice may be referred to as "blood glucose lowering action," and blood glucose suppressing action in OGTT may be referred to as "blood glucose elevation suppressive action."

Blood glucose suppressing activity by OGTT can be determined by measuring blood glucose elevation suppression when mice are force-fed sugar. For example, first, a test compound is administered to mice fasted overnight, and glucose solution is orally administered 30 minutes later. Blood glucose in mice will rise from the administration of glucose, reach maximum at about 30 minutes after administration, and then slowly decrease. The blood glucose suppressing action of the oligosaccharide chain added antigenic GLP-1 analogue can be evaluated by measuring blood glucose 30 minutes after administration of glucose, and comparing it to blood glucose when non-oligosaccharide chain added antigenic GLP-1 analogue is administered.

On the other hand, by comparing the administration dosage of when the same extent of blood glucose elevation suppressive action is confirmed in OGTT, the blood glucose suppressing activity strength of the oligosaccharide chain added antigenic GLP-1 analogue of the present invention can be evaluated. For example, when ten administrations of non-oligosaccharide chain added antigenic GLP-1 analogue and one administration of oligosaccharide chain added antigenic GLP-1 analogue yield the same blood glucose suppressing action, the blood glucose suppressing activity of said oligosaccharide chain added antigenic GLP-1 analogue is 10-fold of the non-oligosaccharide chain added antigenic GLP-1 analogue.

Blood glucose suppressing activity using db/db mice can be determined by measuring blood glucose after administration of a test compound to diabetic mice. Blood glucose after administration of the test compound is measured over time, and if, for example, blood glucose 120 minutes after administration is lower than at the time of administration, blood glucose lowering action can be confirmed. For example, sustainability of blood glucose lowering action can also be determined by measuring blood glucose 300 minutes after administration.

Even if the blood glucose suppressing activity is lower compared to non-oligosaccharide chain added antigenic GLP-1 analogue, increase in blood stability can compensate for this low activity.

For example, among GLP-1 activities, insulin excretion activity can be measured by using in vitro cAMP synthesis capability test etc. GLP-1 increases intracellular cAMP concentration and promotes insulin excretion by binding to the GLP-1 receptor. Accordingly, for example, by stimulating mice GLP-1 receptor-expressing CHO-K1 cells with oligosaccharide chain added antigenic GLP-1 analogues, measuring the amount of cAMP synthesized within cells, and comparing the EC50 to the non-oligosaccharide chain added antigenic GLP-1 analogue, the insulin excretion activity of the oligosaccharide chain added antigenic GLP-1 analogue can be evaluated.

The blood stability of the oligosaccharide chain added antigenic GLP-1 analogue of the present invention is preferably equal to or higher than that of the natural form GLP-1, more preferably equal to or higher than that of the non-oligosaccharide chain added antigenic GLP-1 analogue. Blood stability can be measured using means well-known to those skilled in the art, and for example, plasma stability or resistance to DPP-IV (dipeptidyl peptitase IV) can be measured, and determined using half-life and AUC (area under the curve of blood drug concentration - time) etc. as indicators. Increase in kidney clearance also contributes to increase in blood stability.

The stability of the oligosaccharide chain added antigenic GLP-1 analogue of the present invention in plasma is preferably equal to or higher than that of the natural form GLP-1, more preferably equal to or higher than that of the non-oligosaccharide chain added antigenic GLP-1 analogue.

Resistance to DPP-IV can be determined for example by measuring the half-life in DPP-IV solution. The oligosaccharide chain added antigenic GLP-1 analogue of the present invention is equal to or higher compared to non-oligosaccharide chain added antigenic GLP-1 analogue in resistance to DPP-IV.

The oligosaccharide chain added antigenic GLP-1 analogue of the present invention also has a blood half-life of preferably at least one hour, more preferably at least 3, 5, 7, 10, 15, and 20 hours, and further preferably at least 24 hours.

The oligosaccharide chain added antigenic GLP-1 analogue of the present invention is reduced in its antigenicity compared to the non-oligosaccharide chain added antigenic GLP-1 analogue. Reduction of antigenicity, for example, can be evaluated by sensitizing mice with an oligosaccharide chain added antigenic GLP-1 analogue or non-oligosaccharide chain added antigenic GLP-1 analogue, and measuring blood anti-GLP-1 analogue antibody titer of each mice and comparing them.
The antigenicity of the oligosaccharide chain added antigenic GLP-1 analogue of the present invention is reduced to preferably about half or less, more preferably about one-third or less, and further preferably about one-quarter, compared to the non-oligosaccharide chain added antigenic GLP-1 analogue.

### (Pharmaceutical Composition)

Pharmaceutical compositions containing the oligosaccharide chain added antigenic GLP-1 analogue of the present invention as the active ingredient will now be described.
A pharmaceutical composition containing the oligosaccharide chain added antigenic GLP-1 analogue of the present invention as the active ingredient is effective for therapy or prevention of a GLP-1-related disease. As described above, GLP-1 is known for various actions, and there are various diseases related to these actions. For example, it has been found that glucose intake by cells and reduction in blood glucose is caused by GLP-1 stimulating insulin release. Suppression of gastric and/or intestinal motility, suppression of gastric and/or intestinal content excretion, and suppression of food intake has also been found. Accordingly, GLP-1-related diseases include, e.g., non-insulin-dependent diabetes (NIDDM), insulin-dependent diabetes, cerebral stroke (see International Publication WO 00/16797 by Efendic), myocardial infarction (see International Publication WO 98/08531 by Efendic), obesity (see International Publication WO 98/19698 by Efendic), functional dyspepsia, irritable intestines syndrome (see International Publication WO 99/64060 by Efendic), and islet transplantation. The pharmaceutical composition containing the oligosaccharide chain added antigenic GLP-1 analogue of the present invention as the active ingredient is particularly effective for therapy or prevention of diabetes, and more particularly, it is effective for prevention of type 1 diabetes and therapy of type 2 diabetes.

The above pharmaceutical composition is manufacutred into an ordinary pharmaceutical composition form by employing typically used diluents and excipients such as fillers, bulking agents, binders, wetting agents, disintegrants, surface activaring agents, and lubricants etc.
Such pharmaceutical compositions include, e.g., tablets, pills, powders, solutions, suspensions, emulsions, granules, capsules, suppositories, and injections etc.

The amount of the oligosaccharide chain added antigenic GLP-1 analogue of the present invention contained in the pharmaceutical composition is not particularly limited, and can be appropriately selected from a broad range. It is typically preferable to contain 1 to 70% by weight of the oligosaccharide chain added antigenic GLP-1 analogue of the present invention in the pharmaceutical composition.

The pharmaceutical composition containing the oligosaccharide chain added antigenic GLP-1 analogue of the present invention as the active ingredient can also further contain other active ingredients, and can also be employed in combination with pharmaceutical compositions containing other active ingredients. The pharmaceutical composition containing the oligosaccharide chain added antigenic GLP-1 analogue of the present invention as the active ingredient can also further contain one or more different oligosaccharide chain added antigenic GLP-1 analogues of the present invention as active ingredients, and can also be employed in combination with pharmaceutical compositions containing one or more different oligosaccharide chain added antigenic GLP-1 analogues of the present invention as active ingredients.

The method of administering the pharmaceutical composition according to the present invention is not particularly limited, and it is administered in a method depending on various drug formulation, age and sex of the patient, disease state, and other conditions. The method of administration when it is a tablet, a pill, a solution, a suspension, an emulsion, granules, and a capsule includes e.g. oral administration etc. When it is an injection, it can also be intravenously, intramuscularly, intradermally, subcutaneously, or intraperitoneally administered alone, or mixed with ordinary replacement fluids such as glucose and amino acid. Suppositories are rectally administered.

The administration dosage of the above pharmaceutical composition may be appropriately selected depending on usage, age and sex of the patient, extent of disease, and other conditions, and is typically an administration dosage which will be 0.1 to 900 nmol, preferably 1 to 90 nmol of the oligosaccharide chain added antigenic GLP-1 analogue of the present invention per 1 kg of body weight. Since the oligosaccharide chain added antigenic GLP-1 analogue of the present invention is reduced in antigenicity compared to the non-oligosaccharide chain added antigenic GLP-1 analogue, safety is relatively high even when administration dosage is increased.

The number of administrations of the above pharmaceutical composition may be appropriately selected depending on usage, age and sex of the patient, extent of disease, and other conditions, e.g., three times/day, twice/day, once/day, and further, a less frequent number of administrations (such as once/week and once/month) may also be selected depending on blood stability thereof. Preferably, the number of administrations of the above pharmaceutical composition is once or less per day.

The oligosaccharide chain added to the oligosaccharide chain added antigenic GLP-1 analogue of the present invention is easily degradated by the metabolic system in the body. In one aspect of the present invention, said oligosaccharide chain also has a structure that exists bound to a glycopeptide (or glycoprotein) in vivo. Accordingly, the pharmaceutical composition comprising the oligosaccharide chain added antigenic GLP-1 analogue of the present invention and said peptide as active ingredients do not show adverse effects or antigenicity when administered in vivo, and has advantages of having less concerns for allergic reactions or not being able to gain drug action due to antibody production.

Further, the oligosaccharide chain added antigenic GLP-1 analogue of the present invention can be stably and easily supplied in large amounts, and is very useful also in terms of providing high-quality pharmaceuticals with stable quality.
The present invention also provides a method for therapy or prevention of a GLP-1-related disease, characterized in adminstering an effective amount of the oligosaccharide chain added antigenic GLP-1 analogue of the present invention.

The terms used in herein are employed to describe the particular embodiments, and do not intend to limit the present invention.
The term "comprising", "containing", and "including" as used herein, unless clearly recognized to be different from the context, intends the presence of the described items (such as components, steps, elements, and numbers), and does not exclude the presence of other items (such as components, steps, elements, and numbers).

Unless otherwise defined, all terms (comprising technical and scientific terms) used herein have the same meaning to that broadly recognized by those skilled in the art of the technology to which the present invention belongs. The terms used herein, unless clearly defined otherwise, should be construed as having meanings matching that in the present specification and in related technical fields, and are not to be construed as idealized or excessivly formal meanings.

The embodiments of the present invention may be described referring to schematic diagrams, and in case of schematic diagrams, they may be expressed in exaggeration in order to allow clear description.
Terms such as first and second may be employed to express various elements, although these elements are to be recognized as not being limited by these terms. These terms are used solely for discriminating one element from another, and for example, it is possilbe to describe a first element as a second element, and similarly, to describe a second element as a first element without departing from the scope of the present invention.

The present invention will be described in more detail below referreing to Examples. However, the present invention can be implemented by various aspects, and is not to be construed as limited to the Examples described herein.

### Examples

The present invention will now be specifically described as follows based on Examples, but it is not to be limited thereto in any way.

### Example 1 - Synthesis of Position 30 Cys-DisialoOligosaccharide chain added Exendin-4 (SEQ ID NO: 6)

Ex-4 synthesized by Synthesis Example 1 described below (SEQ ID NO: 10; a 39-residue peptide having Gly at position 30 of the amino acid sequence of Ex-4 set forth in SEQ ID NO: 2 substituted with Cys) 12.0 mg and the bromoacetylated disialooligosaccharide chain shown by the following formula (a) (Otsuka Chemical Co., Ltd.) 36 mg were reacted in 100 mM sodium phosphate buffer pH 7.4 and 5 mM tris-carboxyethylphosphine 1 mL at 37°C for one hour. 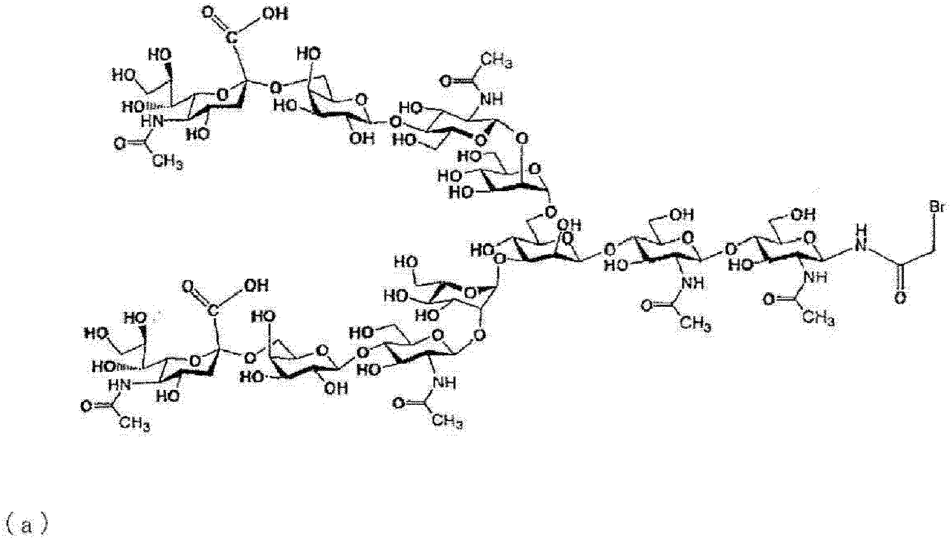 This was directly purified by HPLC [column: SHISEIDO UG-120 (C18, 5 µm), diameter 20 x 250 mm, gradient: solution A: 0.1% TFA-water, solution B: 0.09% TFA/10% water/90% AN, 8 ml/min; solution B 35 -> 50%, 20 min linear gradient], and an oligosaccharide chain added exendin-4 having Gly at position 30 of exendin-4 substituted with a disialooligosaccharide chain added Cys 10.6 mg was obtained. (M: C271H422N58O123S, MALDI TOF Mass calculated for [M+H]⁺ 6493.63, found 6494.33)

### Example 2 - Synthesis of Position 30 Cys-High-Mannose-Type 5-Oligosaccharide chain added Exendin-4 (SEQ ID NO: 7)

Ex-4 synthesized by Synthesis Example 1 described below (SEQ ID NO: 10; a 39-residue peptide having Gly at position 30 of the amino acid sequence of Ex-4 set forth in SEQ ID NO: 2 substituted with Cys) 1.2 mg and the bromoacetylated M5 oligosaccharide chain shown by the following formula (b) synthesized by Synthesis Example 2 described below 3.9 mg were reacted in 35 mM sodium phosphate buffer pH 7.4 and 1 mM tris-carboxyethylphosphine 0.17 mL at 37°C for 3 hours reaction. 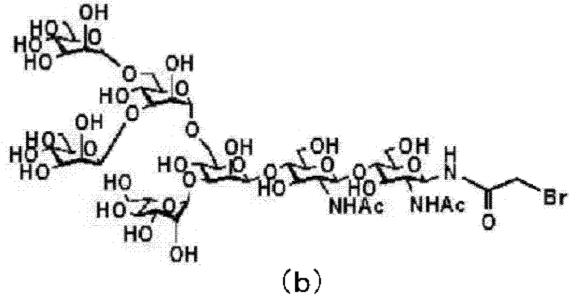 This was directly purified by HPLC [column: SHISEIDO UG-120 (C18, 5 µm), diameter 4.6 x 250 mm, gradient: solution A: 0.1% TFA-water, solution B: 0.09% TFA/10% water/90% AN, 0.7 ml/min; solution B 35 -> 50%, 20 min linear gradient], and an oligosaccharide chain added exendin-4 having Gly at position 30 of exendin-4 substituted with a high-mannose type M5 oligosaccharide chain added Cys 0.5 mg was obtained. (M: C233H362N54O97S, MALDI TOF Mass calculated for [M (average) +H]⁺ 5504.74, found 5506.85)

### Example 3 - Synthesis of Position 30 Cys-AsialoOligosaccharide chain added Liraglutide (SEQ ID NO: 8)

### (1) Synthesis of Position 28 Arg-, Position 30 Cys-GLP-1 (SEQ ID NO: 11)

This was synthesized by peptide solid-phase synthesis by Fmoc method, and purified by HPLC [column: SHISEIDO UG-120 (C18, 5 µm), diameter 20 x 250 mm, gradient: solution A: 0.1% TFA-water, solution B: 0.09% TFA/10% water/90% AN, 8 ml/min; solution B 20 -> 95%, 20 min linear gradient].

### (2) Synthesis of Position 28 Arg-, Position 30 Cys-AsialoOligosaccharide chain added GLP-1 (SEQ ID NO:12)

The synthesized position 28 Arg-, position 30 Cys-GLP-1 (7-37) 21.9 mg, the asialobromoacetyl-oligosaccharide chain shown by the following formula 40.3 mg, and water 1.3 mL were reacted in 100 mM TCEP 60 µL and 200 mM HEPES buffer pH 7.5 700 µL at 37°C for 7 hours. 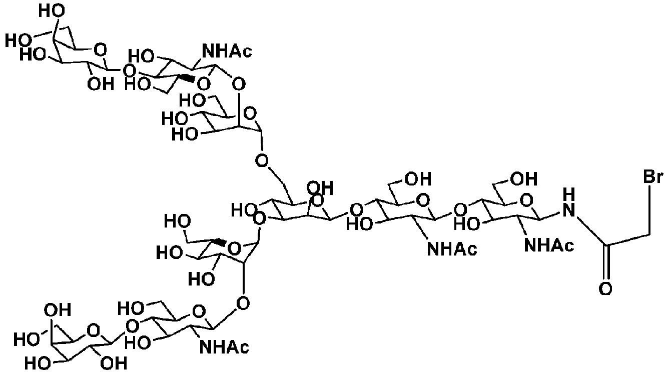 This was directly purified by HPLC [column: SHISEIDO UG-120 (C18, 5 µm), diameter 20 x 250 mm, gradient: solution A: 0.1% TFA-water, solution B: 0.09% TFA/10% water/90% AN, 8 ml/min; solution B 20 -> 95%, 20 min linear gradient], and a position 28 Arg-, position 30 Cys-asialooligosaccharide chain added GLP-1 (7-37) 20.8 mg was obtained.

### (3) Synthesis of Position 30 Cys-AsialoOligosaccharide chain added Liraglutide (SEQ ID NO:8)

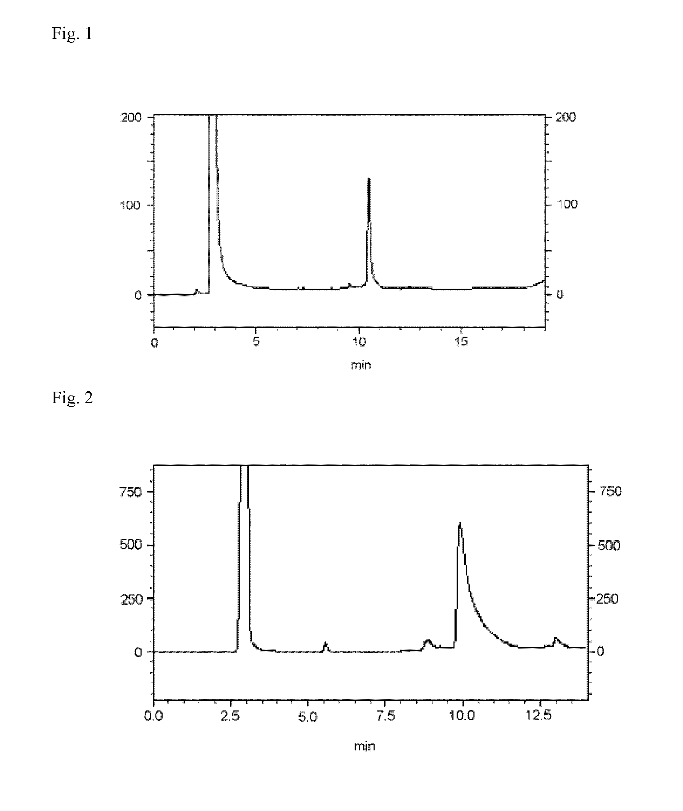

The position 28 Arg-, position 30 Cys-asialooligosaccharide chain added GLP-1 (7-37) synthesized in (2) 7.0 mg and Pal-Glu (OBu)-OSu (see Translation of PCT International Application No. 2002-512175, Example 33) 5.0 mg were reacted in DIPEA 4.6 µL, NMP 300 µL, and water 200 µL for 5 minutes. Aqueous Gly solution (Gly 6 mg, water 100 µL, and EtOH 100 µL) was added, purified by HPLC [column: Zorbax 300 SB-CN, diameter 4.6 x 250 mm, gradient: solution A: 0.1% TFA-water, solution B: 0.09% TFA/10% water/90% AN, 1.0 ml/min; solution B 40 -> 55%, 15 min 65°C linear gradient], and lyophilized (4.3 mg).
Of the obtained lyophilisate, 1.1 mg was treated with TFA, purified again by HPLC with the same conditions (Figure 1), and a 30 Cys asialooligosaccharide chain added liraglutide having Arg at position 30 of liraglutide substituted with asialooligosaccharide chain added Cys 0.8 mg was obtained. (MALDI TOF Mass calculated for [M (average) +H]⁺ 5379.68, found 5378.42)

### Example 4 - Synthesis of Position 30 Cys-DisialoOligosaccharide chain added Liraglutide (SEQ ID NO: 9)

### (1) Synthesis of Position 28 Arg-, Position 30 Cys (acm)-GLP-1 (SEQ ID NO: 13)

This was synthesized by peptide solid-phase synthesis by Fmoc method, and purified by HPLC [column: SHISEIDO UG-120 (C18, 5 µm), diameter 20 x 250 mm, gradient: solution A: 0.1% TFA-water, solution B: 0.09% TFA/10% water/90% AN, 8 ml/min; solution B 20 -> 95%, 20 min linear gradient].

### (2) Synthesis of Position 30 Cys-Liraglutide

The peptide obtained in (1) 10.4 mg and Pal-Glu (OBu)-OSu (see Translation of PCT International Application No. 2002-512175, Example 33) 9.0 mg were reacted in DIPEA 10.9 µL, NMP 600 µL, and water 300 µL for 10 minutes. Aqueous Gly solution (Gly 29 mg, water 400 µL, and EtOH 200 µL) were added, purified by HPLC [column: Zorbax 300 SB-CN, diameter 4.6 x 250 mm, gradient: solution A: 0.1% TFA-water, solution B: 0.09% TFA/10% water/90% AN, 1.0 ml/min; solution B 48-55 (7 min) - 65 (8 min) - 100 (9 min), 15 min 65°C linear gradient], and lyophilized.
Of the obtained peptide 8.2 mg, 5.2 mg was treated with TFA, and then purified by HPLC [column: Zorbax 300 SB-CN, diameter 4.6 x 250 mm, gradient: solution A: 0.1% TFA-water, solution B: 0.09% TFA/10% water/90% AN, 1.0 ml/min; solution B 45-47 (1 min) - 50 (7 min) - 95 (8 min), 14 min 65°C linear gradient], and lyophilized (3.2 mg).
The lyophilisate 3.2 mg was dissolved in 2.5 mM 90% aqueous silver acetate solution (160 µL), and stirred at room temperature for 2 hours. Dithiothreitol (3.8 mg) was added, purified by HPLC [column: Zorbax 300 SB-CN, diameter 4.6 x 250 mm, gradient: solution A: 0.1% TFA-water, solution B: 0.09% TFA/10% water/90% AN, 1.0 ml/min; solution B 45-48 (1 min) - 50 (7 min) - 95 (8 min), 14 min, 65°C linear gradient] (Figure 2), lyophilized, and a peptide having Arg at position 30 of liraglutide substituted with Cys (SEQ ID NO: 14) 2.9 mg was obtained.

### (3) Synthesis of Position 30 Cys-DisialoOligosaccharide chain added Liraglutide (SEQ ID NO:9)

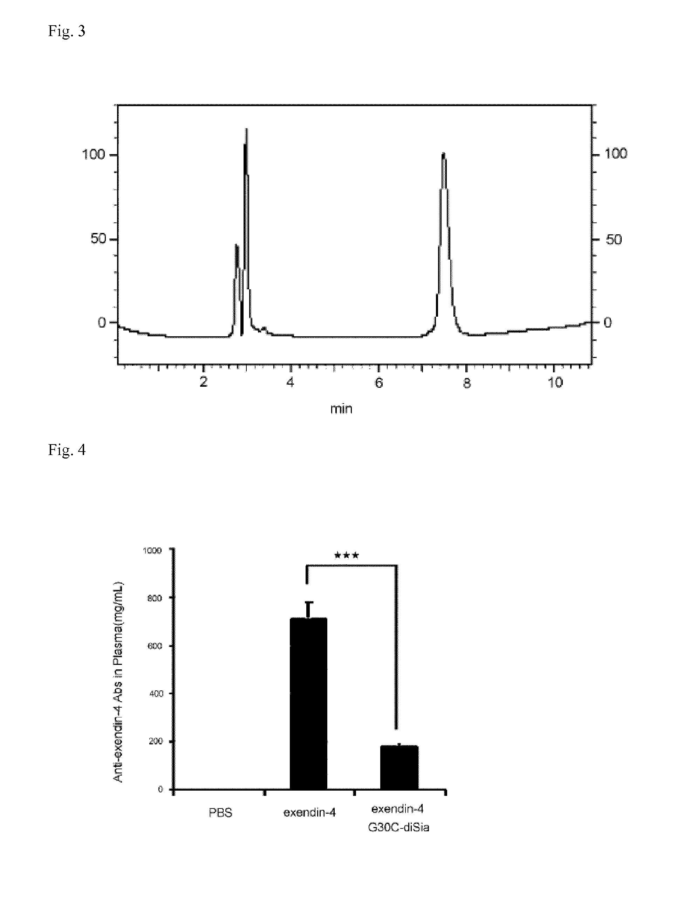

The position 30 Cys-liraglutide obtained in (2) 1.6 mg, the disialobromoacetyl-oligosaccharide chain shown by the following formula 20.0 mg, and water 250 µL were reacted in 100 mM TCEP 40 µL and 200 mM HEPES buffer pH 7.5 480 µL at 37°C for 6 hours. 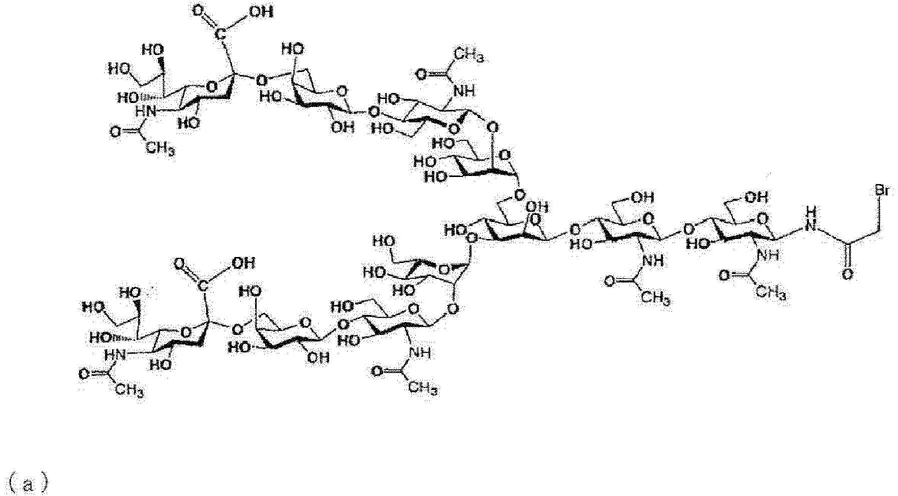 After 9 hours, this was directly purified by HPLC [column: Zorbax 300 SB-CN, diameter 4.6 x 250 mm, gradient: solution A: 0.1% TFA-water, solution B: 0.09% TFA/10% water/90% AN, 1.0 ml/min; solution B 42 - 43 (3 min) - 52 (4 min) - 61 (7 min) - 95 (8 min), 14 min, 65°C linear gradient] (Figure 3), and a position 30 Cys-disialooligosaccharide chain added liraglutide having position 30 Arg of liraglutide substituted with the disialooligosaccharide chain added Cys shown by the following formula 2.3 mg was obtained. (MALDI TOF Mass calculated for [M (average) +H]⁺ 5962.19, found 5963.04) 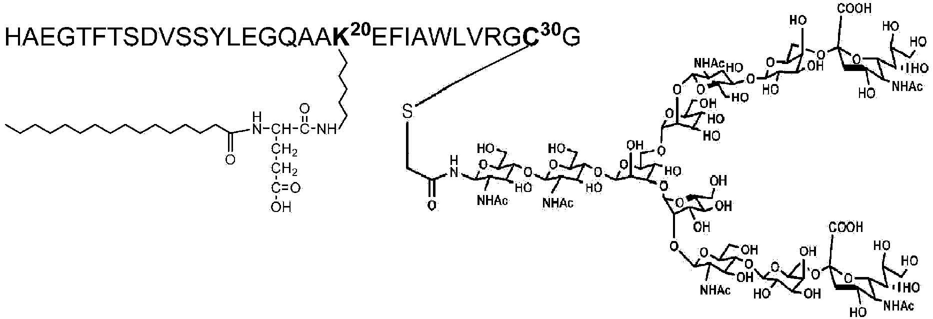 The binding site of the lipophilic substituent to Lys (K²⁰) is as follows.

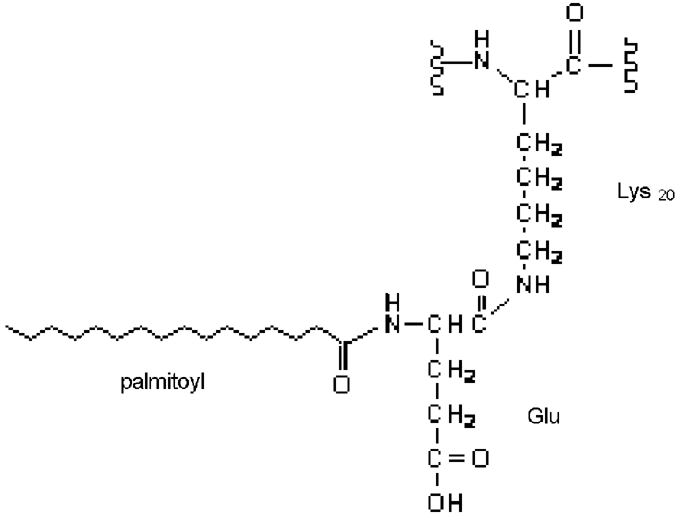

### Example 5 - Synthesis of Position 20 Cys-DisialoOligosaccharide chain added BIM51077 (SEQ ID NO: 21)

A 30-residue peptide having Lys at position 20 of BIM51077 substituted with Cys synthesized in Synthesis Example 3 (SEQ ID NO: 20) (2.4 mg, 0.72 µmol) and guanidine (216 mg) were dissolved in distilled water (240 µL), and aqueous TCEP solution (100 mM, 100 µL), the bromoacetylated disialooligosaccharide chain shown by the following formula (a) (10 mg/mL, 100 µL, 4.26 µmol), and 500 mM sodium phosphate buffer (pH 7.4, 100 µL) were sequentially added. 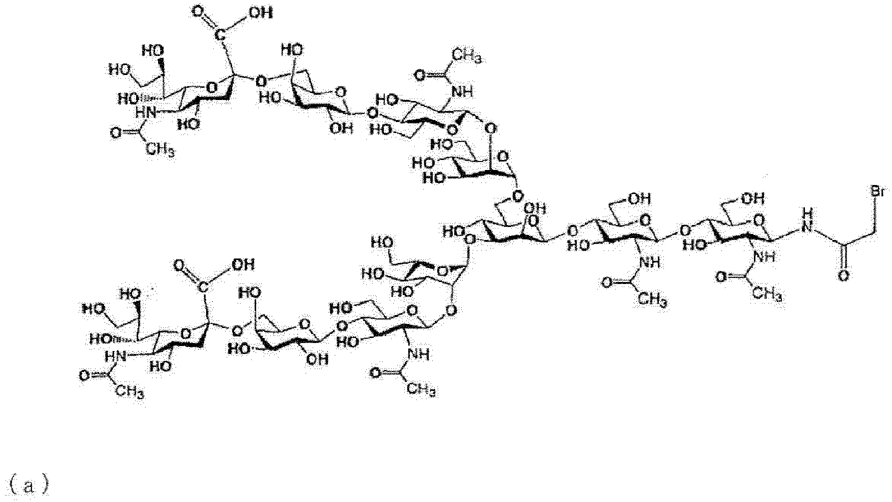 After reacting at 37°C for 2 hours, this was directly purified by HPLC [column: SHISEIDO UG-120 (C18, 5 µm), diameter 4.6 x 250 mm, gradient: solution A: 0.1% TFA-water, solution B: 0.09% TFA/10% water/90% AN, 0.7 ml/min; solution B 35 -> 60%, 20 min lineargradient], and an oligosaccharide chain added BIM51077 peptide having Lys at position 20 of BIM51077 substituted with a disialooligosaccharide chain added Cys (SEQ ID NO: 21) 1.9 mg was obtained. (MALDI TOF Mass calculated for [M (average) +H]+ 5578.72, found 5578.74)

### Comparative Example 1 - Synthesis of Exendin-4 (SEQ ID NO: 2)

After washing the column for solid-phase synthesis with Rink-Amido-PEGA resin (Merck & Co., Inc.) (100 µmol) with DMF, peptide chain elongation was carried out by sequential condensation of amino acids using the method shown below.
An amino acid having the amino group protected with an Fmoc group (0.5mmol) was dissolved in 0.45M HCTU·HOBT/NMP (2.5mmol), added to the column for solid-phase synthesis, and subsequently 0.9M DIPEA/NMP (2.5mmol) was added. After stirring at room temperature for 20 minutes, the resin was washed with DCM and DMF, and the Fmoc group was deprotected with 20% piperidine/DMF solution (2 ml) for 15 minutes. This operation was repeated for sequential condensation of amino acids.
Amino acids protected with Fmoc groups used were Fmoc-Ser (tBu), Fmoc-Pro, Fmoc-Pro, Fmoc-Pro, Fmoc-Ala, Fmoc-Gly, Fmoc-Ser (tBu), Fmoc-Ser (tBu), Fmoc-Pro, Fmoc-Gly, Fmoc-Gly, Fmoc-Asn (Trt), Fmoc-Lys (Boc), Fmoc-Leu, Fmoc-Trp (Boc), Fmoc-Glu (OtBu), Fmoc-Ile, Fmoc-Phe, Fomc-Leu, Fmoc-Arg (Pbf), Fmoc-Val, Fmoc-Ala, Fmoc-Glu (OtBu), Fmoc-Glu (OtBu), Fmoc-Glu (OtBu), Fmoc-Met, Fmoc-Gln (Trt), Fmoc-Lys (Boc), Fmoc-Ser (tBu), Fmoc-Leu, Fmoc-Asp (OtBu), Fmoc-Ser (tBu), Fmoc-Thr (tBu), Fmoc-Phe, Fmoc-Thr (tBu), Fmoc-Gly, Fmoc-Glu (OtBu), Fmoc-Gly, and Fmoc-His (Trt), and a 39-residue peptide of Ser (tBu)-Pro-Pro-Pro-Ala-Gly-Ser (tBu)-Ser (tBu)-Pro-Gly-Gly-Asn (Trt)-Lys (Boc)-Leu-Trp (Boc)-Glu (OtBu)-Ile-Phe-Leu-Arg (Pbf)-Val-Ala-Glu (OtBu)-Glu (OtBu)-Glu (OtBu)-Met-Gln (Trt)-Lys (Boc)-Ser (tBu)-Leu-Asp (OtBu)-Ser (tBu)-Thr (tBu)-Phe-Thr (tBu)-Gly-Glu (OtBu)-Gly-His (Trt) was obtained on the solid-phase resin (SEQ ID NO: 15).
A portion of the obtained resin with the peptide formed thereon was taken in the column for solid-phase synthesis, trifluoroacetic acid:water:TIPS (= 95: 2.5: 2.5) was added so that the resin was sufficiently soaked, and stirred for 3 hours at room temperature. The resin was filtered off, and the reaction solution was concentrated under reduced pressure. The obtained residue was purified by HPLC [column: SHISEIDO UG-120 (C18 5 µm), diameter 20 x 250 mm, gradient: solution A: 0.1% TFA-water, solution B: 0.09% TFA/10% water/90% AN, 8.0 ml/min; solution B 35 -> 60%, 20 min linear gradient], and a 39-residue peptide of Ex-4 (SEQ ID NO: 2) was obtained.

### Comparative Example 2 - Synthesis of GLP-1 (SEQ ID NO: 1)

Amino-PEGA resin (100 µmol) was placed in a column for solid-phase synthesis, and after washing well with DCM and DMF, swelled well with DMF. 4-hydroxymethyl-3-methoxyphenoxy butyric acid (HMPB) (0.25mmol), TBTU (0.25mmol), and N-ethylmorpholine (0.25mmol) were dissolved in DMF (2 ml), placed in the column, and stirred at room temperature for 4 hours. The resin was washed well with DMF and DCM, HMPB-PEGA resin was obtained, and used as the solid-phase for solid-phase synthesis.

Fmoc-Gly (0.50mmol), MSNT (0.50mmol), and N-methylimidazole (0.375mmol) were dissolved in DCM (2 ml), placed in the column for solid-phase synthesis, and stirred at 25°C for 3 hours.

After stirring, the resin was washed with DCM and DMF. The Fmoc group was deprotected with 20% piperidine/DMF solution (2 ml) for 15 minutes. After washing with DMF, subsquent peptide chain elongation was carried out by sequential condensation of amino acids using the method shown below.

An amino acid having the amino group protected with an Fmoc group was dissolved in NMP (1 ml), and after addition of 0.45MHCTU·HOBT/NMP (0.4mmol), added to the column for solid-phase synthesis, and subsequently 0.9MDIPEA/NMP (0.8mmol) was added to the column for solid-phase synthesis. After stirring at room temperature for 20 minutes, the resin was washed with DCM and DMF, and the Fmoc group was deprotected with 20% piperidine/DMF solution (2 ml) for 15 minutes. This operation was repeated, and amino acids protected with Fmoc groups (0.5mmol) were used for sequential condensation of amino acids.

Amino acids protected with Fmoc groups used were Fmoc-Gly, Fmoc-Arg (Pbf), Fmoc-Gly, Fmoc-Lys (Boc), Fmoc-Val, Fmoc-Leu, Fmoc-Trp (Boc), Fmoc-Ala, Fmoc-Ile, Fmoc-Phe, Fmoc-Glu (OtBu), Fmoc-Lys (Boc), Fmoc-Ala, Fmoc-Ala, Fmoc-Gln (Trt), Fmoc-Gly, Fmoc-Glu (OtBu), Fmoc-Leu, Fmoc-Tyr (tBu), Fmoc-Ser (tBu), Fmoc-Ser (tBu), Fmoc-Val, Fmoc-Asp (OtBu), Fmoc-Ser (tBu), Fmoc-Thr (tBu), Fmoc-Phe, Fmoc-Thr (tBu), Fmoc-Gly, Fmoc-Glu (OtBu), Fmoc-Ala, and Fmoc-His (Trt), and a 31-residue peptide of Gly-Arg (Pbf)-Gly-Lys (Boc)-Val-Leu-Trp (Boc)-Ala-Ile-Phe-Glu (OtBu)-Lys (Boc)-Ala-Ala-Gln (Trt)-Gly-Glu (OtBu)-Leu-Tyr (tBu)-Ser (tBu)-Ser (tBu)-Val-Asp (OtBu)-Ser (tBu)-Thr (tBu)-Phe-Thr (tBu)-Gly-Glu (OtBu)-Ala-His (Trt) was obtained on the solid-phase resin (SEQ ID NO: 16).

After washing with DCM and DMF, resin corresponding to 5 µmol of the 31-residue peptide was transferred to an eppendorf tube.

A portion of the obtained resin with the peptide formed thereon was taken in the column for solid-phase synthesis, trifluoroacetic acid:water:TIPS (= 95: 2.5: 2.5) was added so that the resin was sufficiently soaked, and stirred for 3 hours at room temperature. The resin was filtered off, and the reaction solution was concentrated under reduced pressure. The obtained residue was purified by HPLC (Cadenza column C18 100 x 10 mm, developing solvent A: 0.1% TFA-water solution, B: 0.1% TFA acetonitrile:water = 90:10, gradient A:B = 95:5 -> 5:95 15 minutes, flow rate 3.0 ml/min), and GLP-1 (SEQ ID NO: 1) was obtained.

### Comparative Example 3 - Synthesis of Liraglutide (SEQ ID NO: 3)

Preparation of liraglutide was carried out by the following procedure according to the method of Example 34 in Translation of PCT International Application No. JP2002-512175.
This was synthesized by peptide solid-phase synthesis by Fmoc method, and purified by HPLC [column: SHISEIDO UG-120 (C18, 5 µm), diameter 20 x 250 mm, gradient: solution A: 0.1% TFA-water, solution B: 0.09% TFA/10% water/90% AN, 8 ml/min; solution B 20 -> 95%, 20 min linear gradient].
The obtained peptide 10.4 mg and Pal-Glu (OBu)-OSu (9.0 mg) were reacted in DIPEA 10.9 µL, NMP 600 µL, and water 300 µL for 10 minutes. Aqueous Gly solution (Gly 29 mg, water 400 µL, and EtOH 200 µL) was added, purified by HPLC [column: Zorbax 300 SB-CN, diameter 4.6 x 250 mm, gradient: solution A: 0.1% TFA-water, solution B: 0.09% TFA/10% water/90% AN, 1.0 ml/min; solution B 48-55 (7 min) - 65 (8 min) - 100 (9 min), 15 min 65°C ], and then lyophilized.
After treating the obtained protection lipidation peptide with TFA, this was purified by HPLC [column: Zorbax 300 SB-CN, diameter 4.6 x 250 mm, gradient: solution A: 0.1% TFA-water, solution B: 0.09% TFA/10% water/90% AN, 1.0 ml/min; solution B 45-47 (1 min) - 50 (7 min) - 95 (8 min), 14 min, 65°C ], and then lyophilized to obtain liraglutide (SEQ ID NO: 3). (MALDI TOF Mass calculated for [M (average) +H]⁺ 3749.95, found 3750.97)

### Synthesis Example 1 - Synthesis of Peptide Having Position 30 of Exendin-4 Substituted with Cys

After washing the column for solid-phase synthesis with Rink-Amido-PEGA resin (Merck & Co., Inc.) (100 µmol) with DMF, peptide chain elongation was carried out by sequential condensation of amino acids using the method shown below.
An amino acid having the amino group protected with a Fmoc group (0.5mmol) was dissolved in 0.45M HCTU·HOBT/NMP (2.5mmol), added to the column for solid-phase synthesis, and subsequently 0.9M DIPEA/NMP (2.5mmol) was added. After stirring at room temperature for 20 minutes, the resin was washed with DCM and DMF, and Fmoc group was deprotected with 20% piperidine/DMF solution (2 ml) for 15 minutes. This operation was repeated for sequential condensation of amino acids.
Amino acids protected with Fmoc groups used were Fmoc-Ser (tBu), Fmoc-Pro, Fmoc-Pro, Fmoc-Pro, Fmoc-Ala, Fmoc-Gly, Fmoc-Ser (tBu), Fmoc-Ser (tBu), Fmoc-Pro, Fmoc-Cys (Trt), Fmoc-Gly, Fmoc-Asn (Trt), Fmoc-Lys (Boc), Fmoc-Leu, Fmoc-Trp (Boc), Fmoc-Glu (OtBu), Fmoc-Ile, Fmoc-Phe, Fomc-Leu, Fmoc-Arg (Pbf), Fmoc-Val, Fmoc-Ala, Fmoc-Glu (OtBu), Fmoc-Glu (OtBu), Fmoc-Glu (OtBu), Fmoc-Met, Fmoc-Gln (Trt), Fmoc-Lys (Boc), Fmoc-Ser (tBu) Fmoc-Leu, Fmoc-Asp (OtBu), Fmoc-Ser (tBu), Fmoc-Thr (tBu), Fmoc-Phe, Fmoc-Thr (tBu), Fmoc-Gly, Fmoc-Glu (OtBu), Fmoc-Gly, and Fmoc-His (Trt), and a 39-residue peptide of Ser (tBu)-Pro-Pro-Pro-Ala-Gly-Ser (tBu)-Ser (tBu)-Pro-Cys (Trt)-Gly-Asn (Trt)-Lys (Boc)-Leu-Trp (Boc)-Glu (OtBu)-Ile-Phe-Leu-Arg (Pbf)-Val-Ala-Glu (OtBu)-Glu (OtBu)-Glu (OtBu)-Met-Gln (Trt)-Lys (Boc)-Ser (tBu)-Leu-Asp (OtBu)-Ser (tBu)-Thr (tBu)-Phe-Thr (tBu)-Gly-Glu (OtBu)-Gly-His (Trt) was obtained on the solid-phase resin (SEQ ID NO: 17).
A portion of the obtained resin with the peptide formed thereon was taken in the column for solid-phase synthesis, trifluoroacetic acid:water:TIPS (= 95: 2.5: 2.5) was added so that the resin was sufficiently soaked, and stirred for 3 hours at room temperature. The resin was filtered off, and the reaction solution was concentrated under reduced pressure. The obtained residue was purified by HPLC [column: SHISEIDO UG-120 (C18 5 µm), diameter 20 x 250 mm, gradient: solution A: 0.1% TFA-water, solution B: 0.09% TFA/10% water/90% AN, 8.0 ml/min; solution B 35 -> 60%, 20 min linear gradient], and a 39-residue peptide having Gly at position 30 of Ex-4 substituted with Cys was obtained. (MALDI TOF Mass calculated for [M+H]⁺ 4230.60, found 4231.27) (SEQ ID NO: 10)

### Synthesis Example 2 - Synthesis of Bromoacetylated M5 Oligosaccharide chain

Soybean powder 100 g was washed twice with 500 ml of acetone and twice with 500 ml of methanol to obtain delipidated soybean powder 61.4 g.
To the obtained delipidated soybean powder 43.0 g, water 430 ml and liquefying enzyme T (HBI) 4.3 g was added, and reacted at 70°C for 19 hours with stirring. The reaction solution was centrifugated (10000 G, 10 minutes) to separate the supernatant from the precipitate, and supernatant 800 ml was obtained. Water 430 ml and liquefying enzyme T 4.3g were further added to the precipitate, reacted again at 70°C for 19 hours, and the reaction solution was centrifugated (10000 G, 10 minutes) to separate the supernatant from the precipitate, and supernatant 600 ml was obtained.
The obtained supernatants were combined (total 1400 ml), 500 mM phosphate buffer pH 7.0 100 ml and Orientase ONS (HBI) 3.0 g were added, and reacted at 50°C for 19 hours with stirring. The solution after reaction was filtered to remove the insoluble matter, and concentrated on a rotary evaporator until the solution volume was 400 ml. The obtained solution was ultrafiltered using an ultrafilteration membrane having a molecular weight cutoff of 1K (Minimate TFF Capsule 1K membrane, Pall Corporation).
After treatment for 6 hours, the fluid that did not permeate the membrane 230 ml was recovered. To the recovered fluid, 1 M Tris-hydrochloric acid buffer pH 8.0 20 ml, sodium azide 250 mg, and Actinase E (Kaken Pharmaceutical Co., Ltd.) 423.5 mg were added, and reacted at 37°C for 82 hours. The reaction solution was filtered to remove the insoluble matter, and then concentrated on a rotary evaporator until the solution volume was 100 ml. The concentrated solution was fractionated with Sephadex-G25 (diameter 25mm x 100mm) column in two parts each containing half, only the fractions containing the oligosaccharide chain were collected, concentrated, and 2.22 g was obtained.
To the obtained fractions containing the oligosaccharide chain, distilled water 21.0 ml and ethanol 14.9 ml were added and dissolved, sodium bicarbonate 1.13 g and Fmoc-OSu 2.02 g were added, and reacted at room temperature for 16 hours reaction. After the reaction, acetone 250 ml was added, and the precipitate was filtered with a membrane filter (diameter 47mm, retained particle size 0.5 µm, ADVANTEC MFS, INC.). The insoluble matter remaining on the membrane was dissolved in distilled water and recovered, and concentrated on a rotary evaporator until the solution volume was 10 ml or less. The concentrated solution was fractionated with Sephadex-G25 (diameter 25mm x 100mm) column, the fractions containing the oligosaccharide chain were collected, concentrated, and 1.37 g was obtained.
This was further dissolved in distilled water 4 ml, fractionated with ODS column (Wakogel 100 C18, diameter 25mm x 150mm), only the fractions containing the oligosaccharide chain were collected, concentrated, and crude purified oligosaccharide chain 48.6 mg was obtained. The crude purified oligosaccharide chain was purified by HPLC [column: YMC-PackODS-AM diameter 20 x 250 mm, eluent: acetonitrile/25 mM ammonium acetate buffer = 82/18, flow rate : 8.0 ml/min], and a high-mannose Man5GlcNAc₂ oligosaccharide chain (M5 oligosaccharide chain) 13.0 mg was obtained.
To the obtained M5 oligosaccharide chain 11.0 mg, water 165 µl was added and dissolved. To this solution, ammonium bicarbonate 200 mg was added and treated at room temperature for 41 hours, and then lyophilized. To the obtained lyophilisate, sodium bicarbonate 12.5 mg and water 110 µl were added, bromoacetic anhydride (Aldrich) 19.9 mg previously dissolved in 10 µl of N,N-dimethyl formamide (DMF) was added, and reacted for one hour with cooling with ice. After one hour, the reaction system was returned to room temperature, further reacted for one hour, and then purified by gel filteration, and the bromoacetylated M5 oligosaccharide chain (b) shown below 7.9 mg was obtained.

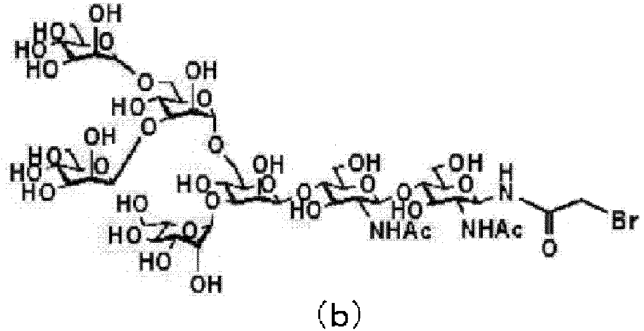

### Synthesis Example 3 - Synthesis of Peptide Having Position 26 of BIM51077 Substituted with Cys

After washing the column for solid-phase synthesis with Rink-Amido-PEGA resin (Merck & Co., Inc.) (100 µmol) with DMF, peptide chain elongation was carried out by sequential condensation of amino acids using the method shown below.

An amino acid having the amino group protected with a Fmoc group (0.5mmol) was dissolved in 0.45M HCTU·HOBT/NMP (2.5mmol), added to the column for solid-phase synthesis, and subsequently 0.9M DIPEA/NMP (2.5mmol) was added. After stirring at room temperature for 20 minutes, the resin was washed with DCM and DMF, and Fmoc group was deprotected with 20% piperidine/DMF solution (2 ml) for 15 minutes. This operation was repeated for sequential condensation of amino acids.

Amino acids protected with Fmoc groups used were Fmoc-Arg (Pbf), Fmoc-Aminoisobutyric Acid (Aib), Fmoc-Lys (Boc), Fmoc-Val, Fmoc-Leu, Fmoc-Trp (Boc), Fmoc-Ala, Fmoc-Ile, Fmoc-Phe, Fmoc-Glu (OtBu), Fmoc-Cys (Trt), Fmoc-Ala, Fmoc-Ala, Fmoc-Gln (Trt), Fmoc-Gly, Fmoc-Glu (OtBu), Fmoc-Leu, Fmoc-Tyr (tBu), Fmoc-Ser (tBu), Fmoc-Ser (tBu), Fmoc-Val, Fmoc-Asp (OtBu), Fmoc-Ser (tBu), Fmoc-Thr (tBu), Fmoc-Phe, Fmoc-Thr (tBu), Fmoc-Gly, Fmoc-Glu (OtBu), Fmoc-Aib, and Fmoc-His (Trt), and a 30-residue peptide of Arg (Pbf)-Aib-Lys (Boc)-Val-Leu-Trp (Boc)-Ala-Ile-Phe-Glu (OtBu)-Cys (Trt)-Ala-Ala-Gln (Trt)-Gly-Glu (OtBu)-Leu-Tyr (tBu)-Ser (tBu)-Ser (tBu)-Val-Asp (OtBu)-Ser (tBu)-Thr (tBu)-Phe-Thr (tBu)-Gly-Glu (OtBu)-Aib-His (Trt) was obtained on the solid-phase resin (SEQ ID NO: 22).

A portion of the obtained resin with the peptide formed thereon was taken in the column for solid-phase synthesis, trifluoroacetic acid:water:TIPS (= 95: 2.5: 2.5) was added so that the resin was sufficiently soaked, and stirred for 3 hours at room temperature. The resin was filtered off, and the reaction solution was concentrated under reduced pressure. The obtained residue was purified by HPLC [column: SHISEIDO UG-120 (C18, 5 µm), diameter 20 x 250 mm, gradient: solution A: 0.1% TFA-water, solution B: 0.09% TFA/10% water/90% AN, 8.0 ml/min; solution B 35 -> 60% 20 min linear gradient], and a 30-residue peptide having Lys at position 20 of BIM51077 substituted with Cys 12 mg was obtained. (MALDI TOF Mass calculated for [M (average) +H]+ 3315.69, found 3314.72) (SEQ ID NO: 20)

### Test Example 1 - Confirmation of Antigenicity-Lowering Action of Exendin-4 by Glycosylation

The effect of glycosylation on the antigenicity of exendin was verified by sensitizing mice with non-oligosaccharide chain added exendin-4 or position 30 Cys-disialooligosaccharide chain added exendin-4, and comparing blood anti-exendin-4 antibody concentrations.
As priming immunization, position 30 Cys-disialooligosaccharide chain added exendin-4 synthesized in Example 1 and non-oligosaccharide chain added exendin-4 (American Peptide Company) were mixed well with Complete Freund's adjuvant (Difco Laboratories) to make emulsions, and intraperitoneally administered to balb/c mice (female, 7 weeks old).
On day 14, oligosaccharide chain added exendin-4 or non-oligosaccharide chain added exendin-4 emulsified by sufficient mixing with Incomplete Freund's adjuvant (Difco Laboratories) were subcutaneously administered to Balb/c mice (female, 7 weeks old) as boost immunization.
Plasma was collected from mice seven days after the boost immunization. The anti-exendin-4 antibody concentration in plasma was measured by Enzyme-Linked Immunosorbent assay (ELISA) using commercially available anti-exendin-4 antibody (Antibodyshop) as the standard.
Fifty µg of non-oligosaccharide chain added exendin-4 was added to a 96-well ELISA plate, and incubated at 37°C for 2 hours. The plate was washed three times with 0.05% Tween 20-containing Phosphate Buffered Saline (PBS), pH 7.4 (wash buffer), and blocked with 0.5% bovine serum albumin-containing wash buffer at 4°C overnight. After the post-blocking plate was washed three times, dilution series prepared by diluting the plasma sample in PBS were added, and incubated at 37°C for 2 hours. The plate was washed three times with wash buffer, 50 µL of peroxidase-conjugated goat anti-mouse IgG was added, and incubated at 37°C for 2 hours. The plate was washed three times with wash buffer, and 100 µL of ABTS peroxidase substrate (Pierce) was added for color development. The color development reaction was terminated by adding 20% SDS, and absorption at 405 nm was measured. The concentration of anti-exendin-4 antibody was determined using anti-exendin-4 monoclonal antibody as the standard. The result is shown in Figure 4.
Antibody production was strongly induced in mice immunized with oligosaccharide chain added and non-oligosaccharide chain added exendin-4, and its concentration in plasma was 707 ± 68.2 mg/mL. In contrast, plasma anti-exendin-4 antibody concentration in mice immunized with position 30-Cys-disialooligosaccharide chain added exendin-4 was reduced to about one-quarter (178.8 ± 22.3 mg/mL) compared to when immunized with non-oligosaccharide chain added exendin-4, showing significant antigenicity-lowering action (p<0.001, Student's t-test).

### Test Example 2 - Oral Glucose Tolerance Test (OGTT) of Oligosaccharide chain added Exendin-4

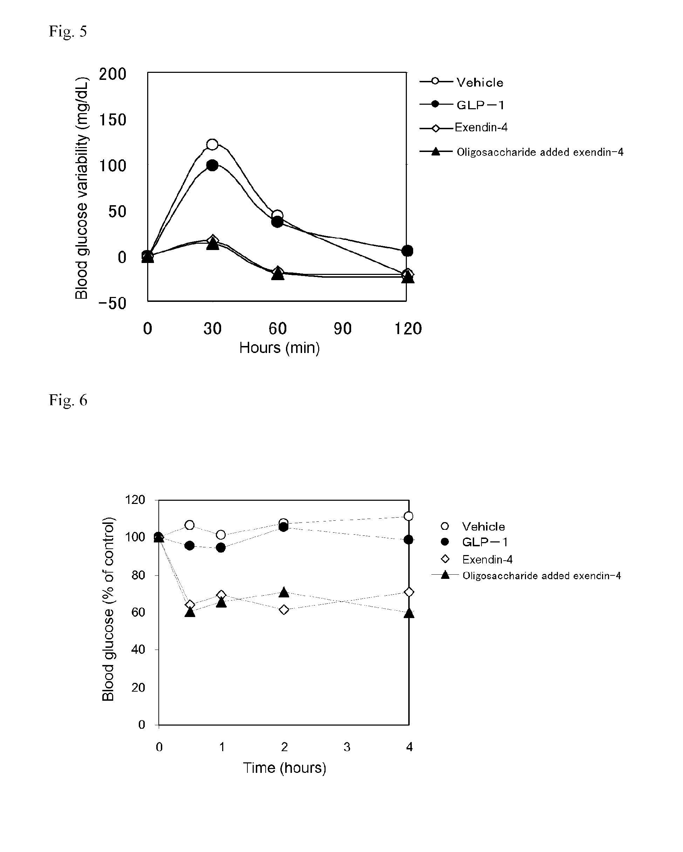

PBS solutions of position 30 Cys-disialooligosaccharide chain added exendin-4 synthesized in Example 1 or non-oligosaccharide chain added exendin-4 synthesized in Comparative Example 1 (0.9 nmol/10 ml), or PBS solution of GLP-1 produced in Comparative Example 2 (9 nmol/10 ml) were intraperitoneally administered to C57BL/6JJc1 mice fasted overnight (10 weeks old, male) at an administration dosage of 10 ml/kg.
Thirty minutes later, glucose solution was orally administered at an administration dosage of 1 mg/g. Orbital blood was collected before administration of glucose, as well as 30 minutes, 60 minutes, and 120 minutes after administration of glucose, and blood glucose was measured using Accu-Chek Aviva (Roche Diagnostics). Similarly, non-oligosaccharide chain added liraglutide was also administered to db/db mice, blood glucose was measured over time, and compared to the action of position 30 Cys-disialooligosaccharide chain added liraglutide. The result is shown in Figure 5.
Position 30 Cys-disialooligosaccharide chain added exendin-4 and non-oligosaccharide chain added exendin-4 showed equal effects, and both showed blood glucose elevation suppressive action higher than vehicle (PBS only) and GLP-1.

### Test Example 3 - Confirmation of Blood Glucose Lowering Action of Oligosaccharide chain added Exendin-4 Using db/db Mice

A PBS solution of position 30 Cys-disialooligosaccharide chain added exendin-4 synthesized in Example 1 (9 nmol/10 mL) was intraperitoneally administered to BKS.Cg-+Leprdb/+Leprdb/Jcl mice (10 weeks old, male) at an administration dosage of 10 mL/kg. Orbital blood was collected before administration of the compound, as well as 30 minutes, 60 minutes, 120 minutes, and 240 minutes later, and blood glucose was measured using Accu-Chek Aviva (Roche Diagnostics).
Blood glucose lowering action was compared with non-oligosaccharide chain added exendin-4 synthesized in Comparative Example 1. The result is shown in Figure 6.
Non-oligosaccharide chain added exendin-4 showed high blood stability, showed strong blood glucose lowering action 30 minutes after administration, and this effect lasted until 240 minutes later. Position 30 Cys-disialooligosaccharide chain added exendin-4 showed blood glucose lowering action and sustainability equal to non-oligosaccharide chain added exendin-4, and no effect of glycosylation of the amino acid at position 30 of exendin-4 was detected.
From the results of Test Examples 1, 2, and 3, glycosylation of position 30 of non-oligosaccharide chain added exendin-4 was thought to be an effective method for reducing antigenicity of non-oligosaccharide chain added exendin-4 without affecting the pharmacologic action of exendin-4.

### Test Example 4 - Oral Glucose Tolerance Test (OGTT) of Oligosaccharide chain added Liraglutide

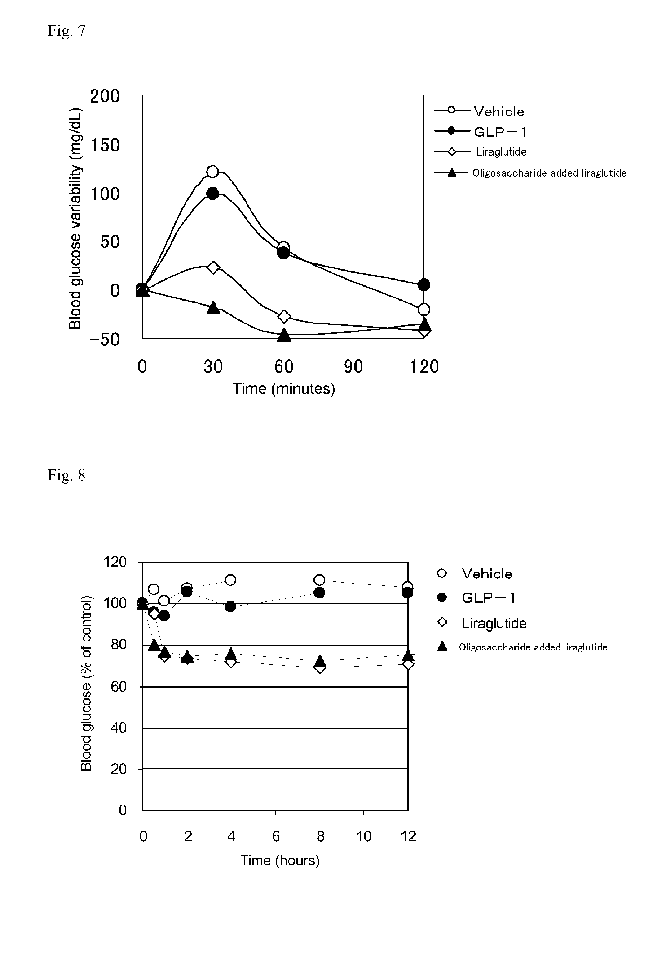

PBS solutions of position 30 Cys-disialooligosaccharide chain added liraglutide produced in Example 4, GLP-1 produced in Comparative Example 2, or liraglutide produced in Comparative Example 3 (9 nmol/10 ml), or PBS solution were intraperitoneally administered to C57BL/6JJc1 mice fasted overnight (10 weeks old, male) at an administration dosage of 10 ml/kg.
Thirty minutes later, glucose solution was orally administered at an administration dosage of 1 mg/g. Orbital blood was collected before administration of glucose, as well as 30 minutes, 60 minutes, and 120 minutes after administration of glucose, and blood glucose was measured using Accu-Chek Aviva (Roche Diagnostics). The result is shown in Figure 7.
Although Position 30 Cys-disialooligosaccharide chain added liraglutide and non-oligosaccharide chain added liraglutide both showed blood glucose elevation suppressive action higher than vehicle (PBS only) and GLP-1, position 30 Cys-disialooligosaccharide chain added liraglutide showed even higher blood glucose elevation suppressive action compared to non-oligosaccharide chain added liraglutide.

### Test Example 5 - Blood glucose elevation Suppressive action Test of Oligosaccharide chain added Liraglutide Using db/db Mice

A PBS solution of position 30 Cys-disialooligosaccharide chain added liraglutide produced in Example 4 (9 nmol/10 mL) was intraperitoneally administered to BKS.Cg-+Leprdb/+Leprdb/Jcl mice (db/db mice, 10 weeks old, male) at an administration dosage of 10 mL/kg. Orbital blood was collected before administration of the compound, as well as 30 minutes, 60 minutes, 120 minutes, 240 minutes, 480 minutes, and 720 minutes later, and blood glucose was measured using Accu-Chek Aviva (Roche Diagnostics). Similarly, non-oligosaccharide chain added liraglutide was also administered to db/db mice, blood glucose was measured over time, and compared to the action of position 30 Cys-disialooligosaccharide chain added liraglutide. The result is shown in Figure 8.
Position 30 Cys-disialooligosaccharide chain added liraglutide showed high blood stability, showed strong blood glucose lowering action 30 minutes after administration, and this effect lasted until 12 hours later. Position 30 Cys-disialooligosaccharide chain added liraglutide showed blood glucose lowering action and sustainability equal to non-oligosaccharide chain added liraglutide, and no effect of glycosylation of the amino acid at position 30 of liraglutide was detected.

### Sequence Listing Free Text

SEQ ID NO: 1 is GLP-1.
SEQ ID NO: 2 is exendin-4.
SEQ ID NO: 3 is liraglutide.
SEQ ID NO: 4 is the general formula of oligosaccharide chain added exendin-4.
SEQ ID NO: 5 is the general formula of oligosaccharide chain added liraglutide.
SEQ ID NO: 6 is exendin-4 having Gly at position 30 substituted with disialooligosaccharide chain added Cys.
SEQ ID NO: 7 is exendin-4 having Gly at position 30 substituted with high-mannose-type 5-oligosaccharide chain added Cys.
SEQ ID NO: 8 is liraglutide having Arg at position 30 substituted with asialooligosaccharide chain added Cys.
SEQ ID NO: 9 is liraglutide having Arg at position 30 substituted with disialooligosaccharide chain added Cys.
SEQ ID NO: 10 is exendin-4 having Gly at position 30 substituted with Cys.
SEQ ID NO: 11 is GLP-1 peptide having Lys at position 28 substituted with Arg, and Arg at position 30 substituted with Cys.
SEQ ID NO: 12 is GLP-1 having Lys at position 28 substituted with Arg, and Arg at position 30 substituted with asialooligosaccharide chain added Cys.
SEQ ID NO: 13 is GLP-1 having having Lys at position 28 substituted with Arg, and Arg at position 30 substituted with Cys (acm).
SEQ ID NO: 14 is liraglutide having Arg at position 30 substituted with Cys.
SEQ ID NO: 15 is a peptide having a protective group synthesized in Comparative Example 1.
SEQ ID NO: 16 is a peptide having a protective group synthesized in Comparative Example 2.
SEQ ID NO: 17 is a peptide having a protective group synthesized in Synthesis Example 1.
SEQ ID NO: 18 is BIM-51077.
SEQ ID NO: 19 is the general formula of oligosaccharide chain added BIM-51077.
SEQ ID NO: 20 is BIM-51077 having Lys at position 20 substituted with Cys.
SEQ ID NO: 21 is BIM-51077 having Lys at position 20 substituted with disialooligosaccharide chain added Cys.
SEQ ID NO: 22 is a peptide having a protective group synthesized in Synthesis Example 3.

## Claims

1. An oligosaccharide chain added form of antigenic GLP-1 analogue having GLP-1 activity, wherein at least one amino acid of the antigenic GLP-1 analogue is substituted with an oligosaccharide chain added amino acid.

2. The oligosaccharide chain added form of antigenic GLP-1 analogue according to claim 1, wherein said oligosaccharide chain added amino acid is an oligosaccharide chain added Asn or an oligosaccharide chain added Cys.

3. The oligosaccharide chain added form of antigenic GLP-1 analogue according to claims 1 or 2, wherein the oligosaccharide chain and the amino acid are bound via a linker in said oligosaccharide chain added amino acid.

4. The oligosaccharide chain added form of antigenic GLP-1 analogue according to any one of claims 1 to 3, wherein said oligosaccharide chain is an oligosaccharide chain consisting of 4 or more sugars.

5. The oligosaccharide chain added form of antigenic GLP-1 analogue according to any one of claims 1 to 4, wherein said oligosaccharide chain is a double-stranded complex oligosaccharide chain.

6. The oligosaccharide chain added form of antigenic GLP-1 analogue according to claim 5, wherein said oligosaccharide chain is an oligosaccharide chain selected from disialooligosaccharide chain, monosialooligosaccharide chain, asialooligosaccharide chain, diGlucNAc oligosaccharide chain, and dimannose oligosaccharide chain.

7. The oligosaccharide chain added form of antigenic GLP-1 analogue according to claim 5, wherein said oligosaccharide chain is an oligosaccharide chain represented by: 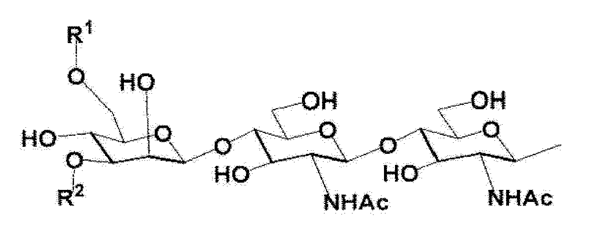 [wherein R¹ and R² are the same or different, and indicate: 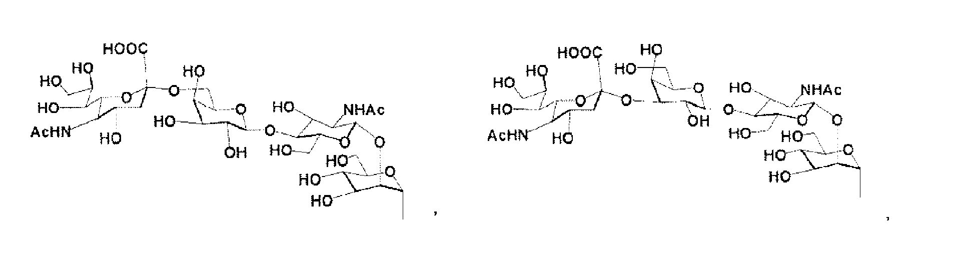 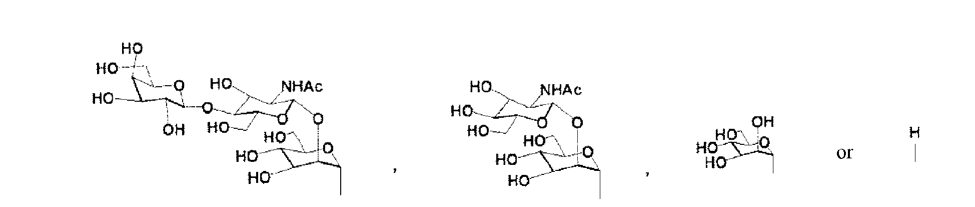 and Ac indicates an acetyl group.]

8. The oligosaccharide chain added form of antigenic GLP-1 analogue according to any one of claims 1 to 7, wherein said antigenic GLP-1 analogue is exendin-4.

9. The oligosaccharide chain added form of antigenic GLP-1 analogue according to claim 8, wherein the site substituted with an oligosaccharide chain added amino acid is position 30 in the amino acid sequence set forth in SEQ ID NO: 2.

10. The oligosaccharide chain added form of antigenic GLP-1 analogue according to claim 8,
wherein in the peptide consisting of the amino acid sequence set forth in SEQ ID NO: 2, Gly at position 30 is substituted with oligosaccharide chain added Cys,
wherein said oligosaccharide chain is the disialooligosaccharide chain shown by the following formula: 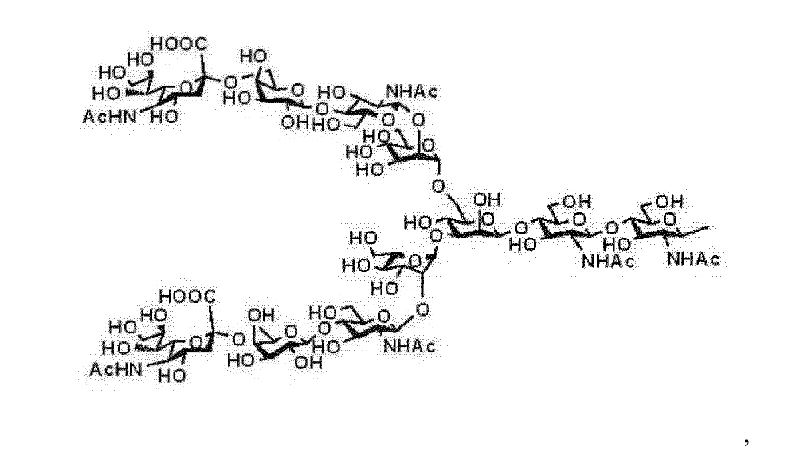 and
wherein said oligosaccharide chain and said Cys are bound via a linker in said oligosaccharide chain added Cys.

11. The oligosaccharide chain added form of antigenic GLP-1 analogue according to any one of claims 1 to 7, wherein said antigenic GLP-1 analogue is liraglutide.

12. The oligosaccharide chain added form of antigenic GLP-1 analogue according to claim 11, wherein said site substituted with an oligosaccharide chain added amino acid is position 30 in the amino acid sequence set forth in SEQ ID NO: 3.

13. The oligosaccharide chain added form of antigenic GLP-1 analogue according to claim 11,
wherein in the peptide consisting of the amino acid sequence set forth in SEQ ID NO: 3, Arg at position 30 is substituted to oligosaccharide chain added Cys,
wherein said oligosaccharide chain is the disialooligosaccharide chain represented by the following formula: 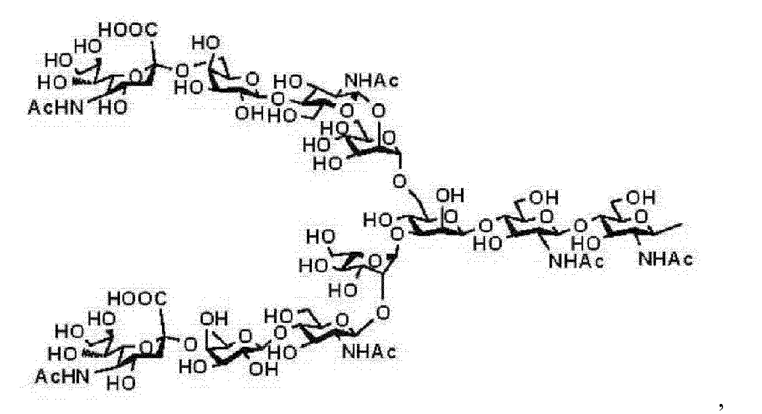 or the asialooligosaccharide chain represented by the following formula: 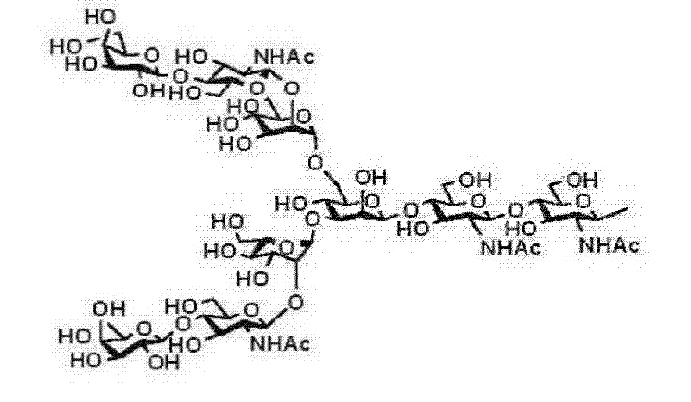 and
wherein said oligosaccharide chain and said Cys are bound via a linker in said oligosaccharide chain added Cys.

14. The oligosaccharide chain added form of antigenic GLP-1 analogue according to any one of claims 1 to 13, wherein said oligosaccharide chain is substantially homogeneous.

15. The oligosaccharide chain added form of antigenic GLP-1 analogue according to any one of claims 1 to 13, wherein said oligosaccharide chain is 99% or more homogeneous.

16. The oligosaccharide chain added form of antigenic GLP-1 analogue according to any one of claims 1 to 15, wherein the antigenicity is half or less relative to an antigenic GLP-1 analogue without any oligosaccharide chains added thereto.

17. The oligosaccharide chain added form of antigenic GLP-1 analogue according to any one of claims 1 to 16, wherein the antigenicity is reduced compared to the antigenic GLP-1 analogue without any oligosaccharide chains added thereto, and GLP-1 activity is elevated compared to natural form GLP-1.

18. A pharmaceutical composition comprising the oligosaccharide chain added form of antigenic GLP-1 analogue according to any one of claims 1 to 17 as an active ingredient.

19. The pharmaceutical composition according to claim 18 for therapy or prevention of a GLP-1-related disease.

20. The pharmaceutical composition according to claim 19, wherein said GLP-1-related disease is diabetes.

21. A therapeutic or prophylactic method of a GLP-1-related disease, **characterized in** adminstering an effective amount of the oligosaccharide chain added form of antigenic GLP-1 analogue according to any one of claims 1 to 17.


SEQUENCE LISTING

<110>  Otsuka Chemical Co., Ltd.

<120>  Glycosylated Product of Antigenic GLP-1 Analog

<130>  OCKP0902F

<150>  JP2009-249597
<151>  2009-10-30

<160>  22

<170>  PatentIn version 3.1


<210>  1
<211>  31
<212>  PRT
<213>  Homo Sapiens

<220>
<223>  GLP-1

<400>  1

His Ala Glu Gly Thr Phe Thr Ser Asp Val Ser Ser Tyr Leu Glu Gly
1               5                   10                  15


Gln Ala Ala Lys Glu Phe Ile Ala Trp Leu Val Lys Gly Arg Gly
            20                  25                  30


<210>  2
<211>  39
<212>  PRT
<213>  Heloderma

<220>
<223>  Exendin-4

<220>
<221>  MOD_RES
<222>  (39)..(39)
<223>  AMIDATION

<400>  2

His Gly Glu Gly Thr Phe Thr Ser Asp Leu Ser Lys Gln Met Glu Glu
1               5                   10                  15


Glu Ala Val Arg Leu Phe Ile Glu Trp Leu Lys Asn Gly Gly Pro Ser
            20                  25                  30


Ser Gly Ala Pro Pro Pro Ser
        35


<210>  3
<211>  31
<212>  PRT
<213>  artificial sequence

<220>
<223>  Liragrutide

<220>
<221>  LIPID
<222>  (20)..(20)
<223>  Lys acylated with palmitic acid via glutamic acid

<400>  3

His Ala Glu Gly Thr Phe Thr Ser Asp Val Ser Ser Tyr Leu Glu Gly
1               5                   10                  15

Gln Ala Ala Lys Glu Phe Ile Ala Trp Leu Val Arg Gly Arg Gly
            20                  25                  30

<210>  4
<211>  39
<212>  PRT
<213>  artificial sequence

<220>
<223>  General formula of glycosylated Exendin-4

<220>
<221>  MISC_FEATURE
<222>  (12)..(12)
<223>  Xaa12: Lys, glycosylated Cys or glycosylated Asn

<220>
<221>  MISC_FEATURE
<222>  (14)..(14)
<223>  Xaa14: Met, glycosylated Cys or glycosylated Asn

<220>
<221>  MISC_FEATURE
<222>  (16)..(16)
<223>  Xaa16: Glu, glycosylated Cys or glycosylated Asn

<220>
<221>  MISC_FEATURE
<222>  (20)..(20)
<223>  Xaa20: Arg, glycosylated Cys or glycosylated Asn

<220>
<221>  MISC_FEATURE
<222>  (24)..(24)
<223>  Xaa24: Glu, glycosylated Cys or glycosylated Asn

<220>
<221>  MISC_FEATURE
<222>  (28)..(28)
<223>  Xaa28: Asn, glycosylated Cys or glycosylated Asn

<220>
<221>  MISC_FEATURE
<222>  (30)..(30)
<223>  Xaa30: Gly, glycosylated Cys or glycosylated Asn

<220>
<221>  MOD_RES
<222>  (39)..(39)

<223>  AMIDATION

<220>
<223>  At least one of Xaa12, Xaa14, Xaa16, Xaa20, Xaa24, Xaa28 and
Xaa30 is glycosylated Cys or glycosylated Asn.

<400>  4

His Gly Glu Gly Thr Phe Thr Ser Asp Leu Ser Xaa Gln Xaa Glu Xaa
1               5                   10                  15


Glu Ala Val Xaa Leu Phe Ile Xaa Trp Leu Lys Xaa Gly Xaa Pro Ser
            20                  25                  30


Ser Gly Ala Pro Pro Pro Ser
        35



<210>  5
<211>  31
<212>  PRT
<213>  artificial sequence

<220>
<223>  General formula of glycosylated Liragrutide

<220>
<221>  MISC_FEATURE
<222>  (12)..(12)
<223>  Xaa12: Ser, glycosylated Cys or glycosylated Asn

<220>
<221>  MISC_FEATURE
<222>  (14)..(14)
<223>  Xaa14: Leu, glycosylated Cys or glycosylated Asn

<220>
<221>  MISC_FEATURE
<222>  (16)..(16)
<223>  Xaa16: Gly, glycosylated Cys or glycosylated Asn

<221>  LIPID
<222>  (20)..(20)
<223>  Lys acylated with palmitic acid via glutamic acid

<220>
<221>  MISC_FEATURE
<222>  (24)..(24)
<223>  Xaa24: Ala, glycosylated Cys or glycosylated Asn

<220>
<221>  MISC_FEATURE
<222>  (28)..(28)
<223>  Xaa28: Arg, glycosylated Cys or glycosylated Asn

<220>
<221>  MISC_FEATURE
<222>  (30)..(30)
<223>  Xaa30: Arg, glycosylated Cys or glycosylated Asn

<220>
<223>  At least one of Xaa12, Xaa14, Xaa16, Xaa24, Xaa28 and Xaa30 is
glycosylated Cys or glycosylated Asn.

<400>  5

His Ala Glu Gly Thr Phe Thr Ser Asp Val Ser Xaa Tyr Xaa Glu Xaa
1               5                   10                  15

Gln Ala Ala Lys Glu Phe Ile Xaa Trp Leu Val Xaa Gly Xaa Gly
            20                  25                  30

<210>  6
<211>  39
<212>  PRT
<213>  artificial sequence

<220>
<223>  Exendin-4 in which Gly(30) is substituted with Cys glycosylated
by disialo oligosaccharide chain

<220>
<221>  CARBOHYD
<222>  (30)..(30)
<223>  Cys glycosylated by disialo oligosaccharide chain

<220>
<221>  MOD_RES
<222>  (39)..(39)
<223>  AMIDATION

<400>  6

His Gly Glu Gly Thr Phe Thr Ser Asp Leu Ser Lys Gln Met Glu Glu
1               5                   10                  15

Glu Ala Val Arg Leu Phe Ile Glu Trp Leu Lys Asn Gly Cys Pro Ser
            20                  25                  30

Ser Gly Ala Pro Pro Pro Ser
        35

<210>  7
<211>  39
<212>  PRT
<213>  artificial sequence

<220>
<223>  Exendin-4 in which Gly(30) is substituted with Cys glycosylated by
high-mannose-5-type oligosaccharide chain

<220>
<221>  CARBOHYD
<222>  (30)..(30)
<223>  Cys glycosylated by high-mannose-5-type oligosaccharide chain

<220>
<221>  MOD_RES
<222>  (39)..(39)
<223>  AMIDATION

<400>  7

His Gly Glu Gly Thr Phe Thr Ser Asp Leu Ser Lys Gln Met Glu Glu
1               5                   10                  15

Glu Ala Val Arg Leu Phe Ile Glu Trp Leu Lys Asn Gly Cys Pro Ser
            20                  25                  30

Ser Gly Ala Pro Pro Pro Ser
        35

<210>  8
<211>  31
<212>  PRT
<213>  artificial sequence

<220>
<223>  Liragrutide in which Arg(30) is substituted with Cys glycosylated
by asialo oligosaccharide chain

<220>
<221>  LIPID
<222>  (20)..(20)
<223>  Lys acylated with palmitic acid via glutamic acid

<220>
<221>  CARBOHYD
<222>  (30)..(30)
<223>  Cys glycosylated by asialo oligosaccharide chain

<400>  8

His Ala Glu Gly Thr Phe Thr Ser Asp Val Ser Ser Tyr Leu Glu Gly
1               5                   10                  15

Gln Ala Ala Lys Glu Phe Ile Ala Trp Leu Val Arg Gly Cys Gly
            20                  25                  30

<210>  9
<211>  31
<212>  PRT
<213>  artificial sequence

<220>
<223>  Liragrutide in which Arg(30) is substituted with Cys
glycosilated by disialo oligosaccharide chain

<220>
<221>  LIPID
<222>  (20)..(20)
<223>  Lys acylated with palmitic acid via glutamic acid

<220>
<221>  CARBOHYD
<222>  (30)..(30)
<223>  Cys glycosylated by disialo oligosaccharide chain

<400>  9

His Ala Glu Gly Thr Phe Thr Ser Asp Val Ser Ser Tyr Leu Glu Gly
1               5                   10                  15

Gln Ala Ala Lys Glu Phe Ile Ala Trp Leu Val Arg Gly Cys Gly
            20                  25                  30

<210>  10
<211>  39
<212>  PRT
<213>  artificial sequence

<220>
<223>  Exendin-4 in which Gly(30) is substituted with Cys

<220>
<221>  MOD_RES
<222>  (39)..(39)
<223>  AMIDATION

<400>  10

His Gly Glu Gly Thr Phe Thr Ser Asp Leu Ser Lys Gln Met Glu Glu
1               5                   10                  15

Glu Ala Val Arg Leu Phe Ile Glu Trp Leu Lys Asn Gly Cys Pro Ser
            20                  25                  30

Ser Gly Ala Pro Pro Pro Ser
        35

<210>  11
<211>  31
<212>  PRT
<213>  artificial sequence

<220>
<223>  GLP-1 in which Lys(28) is substituted with Arg and Arg(30)
is substituted with Cys

<400>  11

His Ala Glu Gly Thr Phe Thr Ser Asp Val Ser Ser Tyr Leu Glu Gly
1               5                   10                  15

Gln Ala Ala Lys Glu Phe Ile Ala Trp Leu Val Arg Gly Cys Gly
            20                  25                  30

<210>  12
<211>  31
<212>  PRT
<213>  artificial sequence

<220>
<223>  GLP-1 in which Lys(28) is substituted with Arg and Arg(30)
is substituted with Cys glycosylated by asialo oligosaccharide chain

<220>
<221>  CARBOHYD
<222>  (30)..(30)
<223>  Cys glycosylated by asialo oligosaccharide chain

<400>  12

His Ala Glu Gly Thr Phe Thr Ser Asp Val Ser Ser Tyr Leu Glu Gly
1               5                   10                  15

Gln Ala Ala Lys Glu Phe Ile Ala Trp Leu Val Arg Gly Cys Gly
            20                  25                  30

<210>  13
<211>  31
<212>  PRT
<213>  artificial sequence

<220>
<223>  GLP-1 in which Lys(28) is substituted with Arg and Arg(30)
is substituted with Cys having blocking group Acm

<220>
<221>  MISC_FEATURE
<222>  (30)..(30)
<223>  Cys having blocking group Acm

<400>  13

His Ala Glu Gly Thr Phe Thr Ser Asp Val Ser Ser Tyr Leu Glu Gly
1               5                   10                  15

Gln Ala Ala Lys Glu Phe Ile Ala Trp Leu Val Arg Gly Cys Gly
            20                  25                  30

<210>  14
<211>  31
<212>  PRT
<213>  artificial sequence

<220>
<223>  Liragrutide in which Arg(30) is substituted with Cys

<220>
<221>  LIPID
<222>  (20)..(20)
<223>  Lys acylated with palmitic acid via glutamic acid

<400>  14

His Ala Glu Gly Thr Phe Thr Ser Asp Val Ser Ser Tyr Leu Glu Gly
1               5                   10                  15

Gln Ala Ala Lys Glu Phe Ile Ala Trp Leu Val Arg Gly Cys Gly
            20                  25                  30

<210>  15
<211>  39
<212>  PRT
<213>  Artificial Sequence

<220>
<223>  Peptide having blocking groups synthesized in Comparative
Example 1

<220>
<221>  MISC_FEATURE
<222>  (1)..(1)
<223>  His having blocking group Trt

<220>
<221>  MISC_FEATURE
<222>  (3)..(3)
<223>  Glu having blocking group OtBu

<220>
<221>  MISC_FEATURE
<222>  (5)..(5)
<223>  Thr having blocking group tBu

<220>
<221>  MISC_FEATURE
<222>  (7)..(7)
<223>  Thr having blocking group tBu

<220>
<221>  MISC_FEATURE
<222>  (8)..(8)
<223>  Ser having blocking group tBu

<220>
<221>  MISC_FEATURE
<222>  (9)..(9)
<223>  Asp having blocking group OtBu

<220>
<221>  MISC_FEATURE
<222>  (11)..(11)
<223>  Ser having blocking group tBu

<220>
<221>  MISC_FEATURE
<222>  (12)..(12)
<223>  Lys having blocking group Boc

<220>
<221>  MISC_FEATURE
<222>  (13)..(13)
<223>  Gln having blocking group Trt

<220>
<221>  MISC_FEATURE
<222>  (15)..(15)
<223>  Glu having blocking group OtBu

<220>
<221>  MISC_FEATURE
<222>  (16)..(16)
<223>  Glu having blocking group OtBu

<220>
<221>  MISC_FEATURE
<222>  (17)..(17)
<223>  Glu having blocking group OtBu

<220>
<221>  MISC_FEATURE
<222>  (20)..(20)
<223>  Arg having blocking group Pbf

<220>
<221>  MISC_FEATURE
<222>  (24)..(24)
<223>  Glu having blocking group OtBu

<220>
<221>  MISC_FEATURE
<222>  (25)..(25)
<223>  Trp having blocking group Boc

<220>
<221>  MISC_FEATURE
<222>  (27)..(27)
<223>  Lys having blocking group Boc

<220>
<221>  MISC_FEATURE
<222>  (28)..(28)
<223>  Asn having blocking group Trt

<220>
<221>  MISC_FEATURE
<222>  (32)..(32)
<223>  Ser having blocking group tBu

<220>
<221>  MISC_FEATURE
<222>  (33)..(33)
<223>  Ser having blocking group tBu

<220>
<221>  MISC_FEATURE
<222>  (39)..(39)
<223>  Ser having blocking group tBu


<400>  15

His Gly Glu Gly Thr Phe Thr Ser Asp Leu Ser Lys Gln Met Glu Glu
1               5                   10                  15


Glu Ala Val Arg Leu Phe Ile Glu Trp Leu Lys Asn Gly Gly Pro Ser
            20                  25                  30


Ser Gly Ala Pro Pro Pro Ser
        35


<210>  16
<211>  31
<212>  PRT
<213>  artificial sequence

<220>
<223>  Peptide having blocking groups synthesized in Comparative
Example 2

<220>
<221>  MISC_FEATURE
<222>  (1)..(1)
<223>  His having blocking group Trt

<220>
<221>  MISC_FEATURE
<222>  (3)..(3)
<223>  Glu having blocking group OtBu

<220>
<221>  MISC_FEATURE
<222>  (5)..(5)
<223>  Thr having blocking group tBu

<220>
<221>  MISC_FEATURE
<222>  (7)..(7)
<223>  Thr having blocking group tBu

<220>
<221>  MISC_FEATURE
<222>  (8)..(8)
<223>  Ser having blocking group tBu

<220>
<221>  MISC_FEATURE
<222>  (9)..(9)
<223>  Asp having blocking group OtBu

<220>
<221>  MISC_FEATURE
<222>  (11)..(11)
<223>  Ser having blocking group tBu

<220>
<221>  MISC_FEATURE
<222>  (12)..(12)
<223>  Ser having blocking group tBu

<220>
<221>  MISC_FEATURE
<222>  (13)..(13)
<223>  Tyr having blocking group tBu

<220>
<221>  MISC_FEATURE
<222>  (15)..(15)
<223>  Glu having blocking group OtBu

<220>
<221>  MISC_FEATURE
<222>  (17)..(17)
<223>  Gln having blocking group Trt

<220>
<221>  MISC_FEATURE
<222>  (20)..(20)
<223>  Lys having blocking group Boc

<220>
<221>  MISC_FEATURE
<222>  (21)..(21)
<223>  Glu having blocking group OtBu

<220>
<221>  MISC_FEATURE
<222>  (25)..(25)
<223>  Trp having blocking group Boc

<220>
<221>  MISC_FEATURE
<222>  (28)..(28)
<223>  Lys having blocking group Boc

<220>
<221>  MISC_FEATURE
<222>  (30)..(30)
<223>  Arg having blocking group Pbf

<400>  16

His Ala Glu Gly Thr Phe Thr Ser Asp Val Ser Ser Tyr Leu Glu Gly
1               5                   10                  15

Gln Ala Ala Lys Glu Phe Ile Ala Trp Leu Val Lys Gly Arg Gly
            20                  25                  30

<210>  17
<211>  39
<212>  PRT
<213>  artificial sequence

<220>
<223>  Peptide having blocking groups synthesized in Synthesis
Example 1

<220>
<221>  MISC_FEATURE
<222>  (1)..(1)
<223>  His having blocking group Trt

<220>
<221>  MISC_FEATURE
<222>  (3)..(3)
<223>  Glu having blocking group OtBu

<220>
<221>  MISC_FEATURE
<222>  (5)..(5)
<223>  Thr having blocking group tBu

<220>
<221>  MISC_FEATURE
<222>  (7)..(7)
<223>  Thr having blocking group tBu

<220>
<221>  MISC_FEATURE
<222>  (8)..(8)
<223>  Ser having blocking group tBu

<220>
<221>  MISC_FEATURE
<222>  (9)..(9)
<223>  Asp having blocking group OtBu

<220>
<221>  MISC_FEATURE
<222>  (11)..(11)
<223>  Ser having blocking group tBu

<220>

<221>  MISC_FEATURE
<222>  (12)..(12)
<223>  Lys having blocking group Boc

<220>
<221>  MISC_FEATURE
<222>  (13)..(13)
<223>  Gln having blocking group Trt

<220>
<221>  MISC_FEATURE
<222>  (15)..(15)
<223>  Glu having blocking group OtBu

<220>
<221>  MISC_FEATURE
<222>  (16)..(16)
<223>  Glu having blocking group OtBu

<220>
<221>  MISC_FEATURE
<222>  (17)..(17)
<223>  Glu having blocking group OtBu

<220>
<221>  MISC_FEATURE
<222>  (20)..(20)
<223>  Arg having blocking group Pbf

<220>
<221>  MISC_FEATURE
<222>  (24)..(24)
<223>  Glu having blocking group OtBu

<220>
<221>  MISC_FEATURE
<222>  (25)..(25)
<223>  Trp having blocking group Boc

<220>
<221>  MISC_FEATURE
<222>  (27)..(27)
<223>  Lys having blocking group Boc

<220>
<221>  MISC_FEATURE
<222>  (28)..(28)
<223>  Asn having blocking group Trt

<220>
<221>  MISC_FEATURE
<222>  (30)..(30)
<223>  Cys having blocking group Trt

<220>
<221>  MISC_FEATURE
<222>  (32)..(32)
<223>  Ser having blocking group tBu

<220>
<221>  MISC_FEATURE
<222>  (33)..(33)
<223>  Ser having blocking group tBu

<220>

<221>  MISC_FEATURE
<222>  (39)..(39)
<223>  Ser having blocking group tBu

<400>  17

His Gly Glu Gly Thr Phe Thr Ser Asp Leu Ser Lys Gln Met Glu Glu
1               5                   10                  15

Glu Ala Val Arg Leu Phe Ile Glu Trp Leu Lys Asn Gly Cys Pro Ser
            20                  25                  30

Ser Gly Ala Pro Pro Pro Ser
        35

<210>  18
<211>  30
<212>  PRT
<213>  artificial sequence

<220>
<223>  BIM51077

<220>
<221>  MISC_FEATURE
<222>  (2)..(2)
<223>  Xaa8: Aib

<220>
<221>  MISC_FEATURE
<222>  (29)..(29)
<223>  Xaa35: Aib

<220>
<221>  MOD_RES
<222>  (30)..(30)
<223>  AMIDATION

<400>  18

His Xaa Glu Gly Thr Phe Thr Ser Asp Val Ser Ser Tyr Leu Glu Gly
1               5                   10                  15

Gln Ala Ala Lys Glu Phe Ile Ala Trp Leu Val Lys Xaa Arg
            20                  25                  30

<210>  19
<211>  30
<212>  PRT
<213>  artificial sequence

<220>
<223>  General formula of glycosylated BIM51077

<220>
<221>  MISC_FEATURE
<222>  (2)..(2)
<223>  Xaa8: Aib

<220>

<221>  MISC_FEATURE
<222>  (12)..(12)
<223>  Xaa18: Ser, glycosylated Cys or glycosylated Asn

<220>
<221>  MISC_FEATURE
<222>  (14)..(14)
<223>  Xaa20: Leu, glycosylated Cys or glycosylated Asn

<220>
<221>  MISC_FEATURE
<222>  (16)..(16)
<223>  Xaa22: Gly, glycosylated Cys or glycosylated Asn

<220>
<221>  MISC_FEATURE
<222>  (20)..(20)
<223>  Xaa26: Lys, glycosylated Cys or glycosylated Asn

<220>
<221>  MISC_FEATURE
<222>  (24)..(24)
<223>  Xaa30: Ala, glycosylated Cys or glycosylated Asn

<220>
<221>  MISC_FEATURE
<222>  (28)..(28)
<223>  Xaa34: Lys, glycosylated Cys or glycosylated Asn

<220>
<221>  MISC_FEATURE
<222>  (29)..(29)
<223>  Xaa35: Aib

<220>
<221>  MISC_FEATURE
<222>  (30)..(30)
<223>  Xaa36: Arg, glycosylated Cys or glycosylated Asn

<220>
<221>  MOD_RES
<222>  (30)..(30)
<223>  AMIDATION

<220>
<223>  At least one of Xaa18, Xaa20, Xaa22, Xaa26, Xaa30, Xaa34 and Xaa36 is
glycosylated Cys or glycosylated Asn.

<400>  19

His Xaa Glu Gly Thr Phe Thr Ser Asp Val Ser Xaa Tyr Xaa Glu Xaa
1               5                   10                  15

Gln Ala Ala Xaa Glu Phe Ile Xaa Trp Leu Val Xaa Xaa Xaa
            20                  25                  30

<210>  20
<211>  30
<212>  PRT
<213>  artificial sequence

<220>
<223>  BIM-51077 in which Lys(20) is substituted with Cys

<220>
<221>  MISC_FEATURE
<222>  (2)..(2)
<223>  Xaa8: Aib

<220>
<221>  MISC_FEATURE
<222>  (29)..(29)
<223>  Xaa35: Aib

<220>
<221>  MOD_RES
<222>  (30)..(30)
<223>  AMIDATION

<400>  20

His Xaa Glu Gly Thr Phe Thr Ser Asp Val Ser Ser Tyr Leu Glu Gly
1               5                   10                  15

Gln Ala Ala Cys Glu Phe Ile Ala Trp Leu Val Lys Xaa Arg
            20                  25                  30

<210>  21
<211>  30
<212>  PRT
<213>  artificial sequence

<220>
<223>  BIM51077 in which Lys(20) is substituted with Cys glycosylated
by disialo oligosaccharide chain

<220>
<221>  MISC_FEATURE
<222>  (2)..(2)
<223>  Xaa8: Aib

<220>
<221>  CARBOHYD
<222>  (20)..(20)
<223>  Cys glycosylated by disialo oligosaccharide chain

<220>
<221>  MISC_FEATURE
<222>  (29)..(29)
<223>  Xaa35: Aib

<220>
<221>  MOD_RES
<222>  (30)..(30)
<223>  AMIDATION

<400>  21

His Xaa Glu Gly Thr Phe Thr Ser Asp Val Ser Ser Tyr Leu Glu Gly
1               5                   10                  15

Gln Ala Ala Cys Glu Phe Ile Ala Trp Leu Val Lys Xaa Arg
            20                  25                  30

<210>  22
<211>  30
<212>  PRT
<213>  artificial sequence

<220>
<223>  amino acid sequence having blocking groups (Synthetic Example 3)

<220>
<221>  MISC_FEATURE
<222>  (1)..(1)
<223>  His having blocking group Trt

<220>
<221>  MISC_FEATURE
<222>  (2)..(2)
<223>  Xaa8: Aib

<220>
<221>  MISC_FEATURE
<222>  (3)..(3)
<223>  Glu having blocking group OtBu

<220>
<221>  MISC_FEATURE
<222>  (5)..(5)
<223>  Thr having blocking group tBu

<220>
<221>  MISC_FEATURE
<222>  (7)..(7)
<223>  Thr having blocking group tBu

<220>
<221>  MISC_FEATURE
<222>  (8)..(8)
<223>  Ser having blocking group tBu

<220>
<221>  MISC_FEATURE
<222>  (9)..(9)
<223>  Asp having blocking group OtBu

<220>
<221>  MISC_FEATURE
<222>  (11)..(11)
<223>  Ser having blocking group tBu

<220>
<221>  MISC_FEATURE
<222>  (12)..(12)
<223>  Ser having blocking group tBu

<220>
<221>  MISC_FEATURE
<222>  (13)..(13)
<223>  Tyr having blocking group tBu

<220>
<221>  MISC_FEATURE
<222>  (15)..(15)
<223>  Glu having blocking group OtBu

<220>
<221>  MISC_FEATURE

<222>  (17)..(17)
<223>  Gln having blocking group Trt

<220>
<221>  MISC_FEATURE
<222>  (20)..(20)
<223>  Cys having blocking group Trt

<220>
<221>  MISC_FEATURE
<222>  (21)..(21)
<223>  Glu having blocking group OtBu

<220>
<221>  MISC_FEATURE
<222>  (25)..(25)
<223>  Trp having blocking group Boc

<220>
<221>  MISC_FEATURE
<222>  (28)..(28)
<223>  Lys having blocking group Boc

<220>
<221>  MISC_FEATURE
<222>  (29)..(29)
<223>  Xaa35: Aib

<220>
<221>  MISC_FEATURE
<222>  (30)..(30)
<223>  Arg having blocking group Pbf

<220>
<221>  MOD_RES
<222>  (30)..(30)
<223>  AMIDATION

<400>  22

His Xaa Glu Gly Thr Phe Thr Ser Asp Val Ser Ser Tyr Leu Glu Gly
1               5                   10                  15

Gln Ala Ala Cys Glu Phe Ile Ala Trp Leu Val Lys Xaa Arg
            20                  25                  30